# FASCICULE DE BREVET EUROPEEN

(11) **EP 1 638 943 B1**
(45) Date de publication et mention de la délivrance du brevet: **06.08.2008**
(21) Numéro de dépôt: 04767175.5
(22) Date de dépôt: 26.05.2004
(51) Int. Cl.: C07D 233/64, A61K 31/4164, A61P 35/00, C07D 403/10, C07D 405/10, C07D 409/10

(54) **NOUVEAUX DERIVES D’IMIDAZOLES, LEUR PREPARATION ET LEUR UTILISATION EN TANT QUE MEDICAMENT**
NEUE IMIDAZOLDERIVATIVE, DEREN HERSTELLUNG UND DEREN VERWENDUNG ALS MEDIKAMENT
NOVEL IMIDAZOLE DERIVATIVES, THE PRODUCTION THEREOF, AND THE USE OF THE SAME AS A MEDICAMENT

(30) Priorité: 27.05.2003 FR 0306403; 04.06.2003 FR 0306712
(43) Date de publication de la demande: 29.03.2006
(73) Titulaire: Société de Conseils de Recherches et d'Applications Scientifiques ( S.C.R.A.S.), 75016 Paris (FR)
(72) Inventeur: BIGG, Dennis, F-91190 Gif-sur-Yvette (FR); LIBERATORE, Anne-Marie, F-78610 Auffargis (FR); PONS, Dominique, F-75014 Paris (FR)
(74) Mandataire: Bourgouin, André
(86) Numéro de dépôt international: PCT/FR2004/001297
(87) Numéro de publication internationale: WO 2004/106307

(56) Documents cités:
- WO-A-99/02155
- LE WANG, K W WOODS, Q LI, K J BARR, R. W. MCCROSKEY, S. M. HANNICK, L. GHERKE, R B CREDO, Y-H HUI, K MARSH, R WARNER, ET AL: "Potent, orally, active heterocycle-based combretastatin A-4 analogues : synthesis, structure-activity relationship, pharmacokinetics, and in vivo antitumor activity evaluation" J. MED. CHEM., vol. 45, 2002, pages 1697-1711, XP002304485

## Description

La présente demande a pour objet de nouveaux dérivés d'imidazoles. L'invention concerne également des compositions pharmaceutiques contenant ces dérivés et leur utilisation pour la préparation d'un médicament.

Les dérivés d'imidazoles selon la présente invention présentent une activité anti-tumorale et en particulier une activité inhibitrice de la polymérisation de la tubuline.

Cible de plusieurs médicaments anticancéreux, la tubuline est une protéine de petite taille qui, en se polymérisant, donne les microtubules du fuseau achromatique qui permet la division cellulaire lors de la mitose. Les vinca-alcaloïdes inhibent sa polymérisation, alors que le paclitaxel et le docétaxel la stabilisent exagérément. Dans les deux cas, la mitose ne peut se dérouler normalement, ce qui entrave la prolifération cellulaire.

En raison de leur activité anti-tumorale, les composés selon l'invention peuvent être utilisés pour le traitement de tumeurs ou de cancers comprenant les cancers de l'oesophage, de l'estomac, des intestins, du rectum, de la cavité orale, du pharynx, du larynx, du poumon, du colon, du sein, du cervix uteri, du corpus endométrium, des ovaires, de la prostate, des testicules, de la vessie, des reins, du foie, du pancréas, des os, des tissus conjonctifs, de la peau, des yeux, du cerveau, les mélanomes et les cancers du système nerveux central, ainsi que le cancer de la thyroïde, la leucémie, la maladie de Hodgkin, les lymphomes autres que ceux de Hodgkin, les myélomes multiples et autres. Par ailleurs ces composés pourraient être également utilisés à traiter certaines infections virales telles que le syndrome immunodifficitaire acquis, l'hépatite C ainsi que des maladies autoimmunes et certaines maladies dégénératives.

L'invention a donc pour objet un composé de formule générale (I) 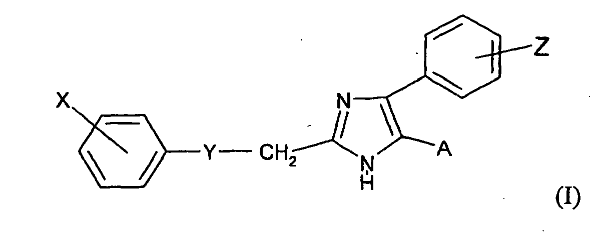 sous forme racémique, d'énantiomère ou toutes combinaisons de ces formes et dans laquelle :
X représente un ou plusieurs substituants identiques ou différents choisis parmi H et halo ;
Y représente -O- ou -S- ;
A représente H ou (C₁-C₆)alkyle ;
Z représente un ou plusieurs substituants identiques ou différents choisis parmi :
   - (C₁-C₆)alkyle éventuellement substitué par un ou plusieurs radicaux halo identiques ou différents ;
   - aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, -(CH₂)ₙ-NR₃R₄, (C₁-C₆)alkyl-sulfonyle, (C₁-C₆)alkyl-thio, (C₁-C₆)alkoxy éventuellement substitué par un ou plusieurs radicaux halo identiques ou différents, (C₁-C₆)alkoxy-carbonyle, phosphate, sulfate, glycoside et -NH-C(O)-CH(R_{A})-NR₅R₆;
   - aryl-(C₁-C₆)alkyle;
   - hétéroaryle;
   - -Z₁-Z'₁;
   - -NR_{N}-C(O)-Z'₂; ou
   - -Z₂-Z'₂;
Z₁ représente -O- ,-C(O)-O-, -NR_{N}-C(O)- ou -C(O)-NR_{N}- ;
Z'₁ représente un radical (C₁-C₁₀)alkyle; aryle-(C₁-C₆)alkyle dont le radical aryle est éventuellement substitué par un ou plusieurs radicaux halo identiques ou différents ; ou (C₁-C₆)alkyle substitué par un ou plusieurs substituants choisis parmi : halo, (C₁-C₆)alkoxy, (C₁-C₆)alkylthio et -NR₁R₂ ;
R₁ et R₂ représentent, indépendamment, H ou (C₁-C₆)alkyle, ou bien forment ensemble avec l'atome d'azote sur lequel ils sont rattachés, un hétérocycloalkyle éventuellement substitué par (C₁-C₆)alkyle ;
Z₂ représente -O-, -S-, -SO₂-, -C(O)-, -C(O)-NR_{N}- ou NR_{N}- ;
Z'₂ représente un radical aryle ou hétéroaryle, les radicaux aryle et hétéroaryle étant éventuellement substitués par un ou plusieurs radicaux identiques ou différents choisis parmi :
   halo, nitro, cyano, hydroxy, (C₁-C₆)alkyle éventuellement substitué par un ou plusieurs radicaux halo identiques ou différents, (C₁-C₆)alkyl-thio, (C₁-C₆)alkyl-sulfonyle, (C₁-C₆)alkoxy éventuellement substitué par un ou plusieurs radicaux halo identiques ou différents, aryl-alkoxy, (C₁-C₆)alkoxy-carbonyle, phosphate, sulfate, glycoside, -(CH₂)ₙ-NR₃R₄ et -NH-C(O)-CH(R_{A})-NR₅R₆ ;
R₃ et R₄ représentent, indépendamment, H, (C₁-C₆)alkyle, (C₁-C₆)alkyl-carbonyle ou (C₁-C₆)alkyl-sulfonyle, ou bien R₃ et R₄ forment ensemble avec l'atome d'azote sur lequel ils sont rattachés, un hétéroaryle ou un hétérocycloalkyle éventuellement substitué par (C₁-C₆)alkyle ;
R₅ et R₆ représentent, indépendamment, H ou (C₁-C₆)alkyle ;
R_{A} représente le résidu associé à l'acide aminé de formule NH₂-CH(R_{A})-C(O)-OH ;
R_{N} représente l'hydrogène ou un radical (C₁-C₆)alkyle ;
n représente un entier de 0 à 3 ; ou un sel pharmaceutiquement acceptable de ce dernier,
à l'exclusion des composés dans lesquels A représente l'atome d'hydrogène et Z le radical-3-CF₃.

L'invention couvre bien évidemment toutes les formes tautomères des composés de formule (I) tels que définis ci-dessus.

Dans les définitions indiquées ci-dessus, l'expression halo (halogéno) représente le radical fluoro, chloro, bromo ou iodo, de préférence fluoro, chloro ou bromo. L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, néopentyle, 2,2-diméthyl-propyle, hexyle, isohexyle ou 1,2,2-triméthyl-propyle. Le terme (C₄-C₁₀)alkyle désigne un radical alkyle ayant de 4 à 10 atomes de carbone, linéaire ou ramifié, tels les radicaux contenant de 4 à 6 atomes de carbone tels que définis ci-dessus mais également heptyle, octyle, 1,1,2,2-tétraméthyl-propyle, 1,1,3,3-tétraméthyl-butyle, nonyle ou décyle. Le terme (C₁-C₂₀)alkyle désigne un radical alkyle ayant de 1 à 20 atomes de carbone, linéaire ou ramifié, tels les radicaux contenant de 1 à 10 atomes de carbone tels que définis ci-dessus mais également les radicaux contenant de 11 à 20 atomes de carbone tels que undécyle, dodécyle, tridécyle, tétradécyle, pentadécyle, hexadécyle, heptadécyle, octadécyle, nonadécyle ou eicosanyle.

L'expression alkyl-sulfonyle représente de préférence un radical dans lequel le radical alkyle est tel que défini ci-dessus comme par exemple, méthylsulfonyle, éthylsulfonyle. De même l'expression alkyl-carbonyle représente de préférence un radical dans lequel le radical alkyle est tel que défini ci-dessus comme par exemple méthylcarbonyle, éthylcarbonyle. Le terme (C₁-C₆)alkylthio désigne les radicaux dans lesquels le radical alkyle est tel que défini ci-dessus comme par exemple les radicaux méthylthio, éthylthio.

Le terme (C₁-C₆)alkoxy désigne les radicaux dans lesquels le radical alkyle est tel que défini ci-dessus comme par exemple les radicaux méthoxy, éthoxy, propyloxy ou isopropyloxy mais également butoxy linéaire, secondaire ou tertiaire, pentyloxy. Le terme alkoxy-carbonyle désigne de préférence les radicaux dans lesquels le radical alkoxy est tel que défini ci-dessus comme par exemple méthoxycarbonyle, éthoxycarbonyle.

L'expression aryle représente un radical aromatique, constitué d'un cycle ou de cycles condensés, comme par exemple le radical phényle, naphtyle ou fluorényle. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux pyrrolyle, imidazolyle, pyrazolyle, isothiazolyle, thiazolyle, isoxazolyle, oxazolyle, triazolyle, thiadiazolyle, pyridyle, pyrazinyle, pyrimidyle, quinolyle, isoquinolyle, quinoxalinyle, indolyle, benzoxadiazoyle, carbazolyle, purinyle, triazinyle, pyrrazolo-pyrimidyle mais également thiényle, benzothiényle, furyle, benzofuryle ou pyranyle. Le terme aralkyle (arylalkyle) désigne de préférence les radicaux dans lesquels les radicaux aryle et alkyle sont tels que définis ci-dessus comme par exemple benzyle ou phénéthyle. Le terme arylalkoxy désigne de préférence les radicaux dans lesquels les radicaux aryle et alkoxy sont tels que définis ci-dessus comme par exemple benzyloxy ou phényléthoxy.

L'expression hétérocycloalkyle désigne un système saturé monocyclique ou bicyclique condensé contenant de 2 à 7 atomes de carbone et au moins un hétéroatome. Ce radical peut contenir plusieurs hétéroatomes identiques ou différents. De préférence, les hétéroatomes sont choisis parmi l'oxygène, le soufre ou l'azote. Comme exemple d'hétérocycloalkyle contenant au moins un atome d'azote, on peut citer les cycles pyrrolidine, imidazolidine, pyrrazolidine, isothiazolidine, thiazolidine, isoxazolidine, oxazolidine, pipéridine, pipérazine, azépane (azacycloheptane), azacyclooctane, diazépane, morpholine, décahydroisoquinoline (ou décahydroquinoline).

L'expression phosphate représente le radical de formule -OP(O)(ORₚ')(ORₚ") dans laquelle Rₚ' et Rₚ" désignent, indépendamment, un radical choisi parmi : H, (C₁-C₂₀)alkyle linéaire et ramifié, aryle et arylalkyle.

L'expression sulfate représente le radical de formule -OS(O)₂(OR) dans lequel R désigne un radical choisi parmi : H, (C₁-C₂₀)alkyle linéaire ou ramifié, aryle et arylalkyle.

L'expression glycoside représente des radicaux tels que les radicaux glucosyles, maltosyles, glucuronyles.

Dans la présente demande, R_{A} représente le résidu associé à l'acide aminé de formule NH₂-CH(R_{A})-C(O)-OH. De préférence, R_{A} représente le radical R_{AA} associé aux acides aminés naturels de formule NH₂-CH(R_{AA})-C(O)-OH que sont la glycine, l'alanine, la valine, l'isoleucine, la leucine, la méthionine, la phénylalanine, la tyrosine, le tryptophane, la sérine, la thréonine, l'asparagine, la glutamine, l'acide aspartique, l'acide glutamique, l'histidine, la lysine, l'arginine, la cystéine et la proline.

Dans la présente demande également, le radical (CH₂)ₙ représente une chaîne hydrocarbonée, linéaire ou ramifiée, de n atomes de carbone.

Selon la présente demande également, lorsqu'un radical a pour formule -B-D-E avec D représentant par exemple -C(O)-NH-, cela signifie que l'atome de carbone de -C(O)-NH- est lié à B et l'atome d'azote à E.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, benzènesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

De préférence, l'invention concerne un composé de formule (I) tel que défini ci-dessus et caractérisé en ce que
X représente un ou plusieurs substituants identiques ou différents choisis parmi H et halo ;
Y représente -O- ou -S- ;
A représente H ou (C₁-C₆)alkyle ;
Z représente un ou plusieurs substituants identiques ou différents choisis parmi :
   - (C₁-C₆)alkyle éventuellement substitué par un ou plusieurs radicaux halo identiques ou différents ;
   - aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
   - hétéroaryle ;
   - -Z₁-Z'₁ ;
   - -NH-C(O)-Z'₂ ; ou
   - -Z₂-Z'₂;
Z₁ représente -O-, -NH-C(O)- ou -C(O)-NH- ;
Z'₁ représente un radical (C₄-C₁₀)alkyle ; aryle-(C₁-C₆)alkyle dont le radical aryle est éventuellement substitué par un ou plusieurs radicaux halo identiques ou différents ; ou (C₁-C₆)alkyle substitué par un ou plusieurs substituants choisis parmi halo, (C₁-C₆)alkoxy, (C₁-C₆)alkylthio et -NR₁R₂;
R₁ et R₂ représentent, indépendamment, H ou (C₁-C₆)alkyle, ou bien forment ensemble avec l'atome d'azote sur lequel ils sont rattachés, un hétérocycloalkyle éventuellement substitué par (C₁-C₆)alkyle;
Z₂ représente -O-, -S-, -SO₂-, -C(O)- ou -C(O)-NH-;
Z'₂ représente un radical aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ; ou un sel pharmaceutiquement acceptable de ce dernier.

De préférence, l'invention concerne un composé de formule (I) tel que défini ci-dessus et caractérisé en ce que A représente H et Y représente -O- ; ou un sel pharmaceutiquement acceptable de ce dernier.

De préférence, l'invention concerne un composé de formule (I) tel que défini ci-dessus et caractérisé en ce que X représente H ; ou un sel pharmaceutiquement acceptable de ce dernier.

De préférence, l'invention concerne un composé de formule (I) tel que défini ci-dessus et caractérisé en ce que
Z représente un ou plusieurs substituants, identiques ou différents, en position méta et/ou para et choisis parmi hétéroaryle et -Z₂-Z'₂;
Z₂ représente -O-, -S-, -SO₂-, -C(O)- ou -C(O)-NH-;
Z'₂ représente l'un des radicaux phényle ou naphtyle éventuellement substitués par un ou plusieurs radicaux identiques ou différents choisis parmi halo, nitro, cyano, hydroxy, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ; ou un sel pharmaceutiquement acceptable de ce dernier.

De préférence également, l'invention concerne un composé de formule (I) tel que défini ci-dessus et caractérisé en ce que Z représente
- un hétéroaryle ;
- Z₁-Z'₁ dans lequel
   soit Z₁ représente -O-, -NR_{N}-C(O)- ou -C(O)-NR_{N}- et Z'₁ représente le radical benzyle ;
   soit Z₁ représente -O-, -C(O)-O-, -NR_{N}-C(O)- ou -C(O)-NR_{N}- et Z'₁ représente un radical (C₁-C₆)alkyle substitué par un ou plusieurs substituants choisis parmi : halo, (C₁-C₆)alkoxy, (C₁-C₆)alkylthio et -NR₁R₂ ;
   R₁ et R₂ représentent, indépendamment, H ou (C₁-C₆)alkyle, ou bien forment ensemble avec l'atome d'azote sur lequel ils sont rattachés, un hétérocycloalkyle ;
- -Z₂-Z'₂ dans lequel
   Z₂ représente -O-, -S-, -SO₂-, -C(O)-, -C(O)-NR_{N}- ou NR_{N}- ;
   Z'₂ représente un radical phényle ou phényle substitué par un ou plusieurs radicaux identiques ou différents choisis parmi :
      halo, nitro, cyano, hydroxy, (C₁-C₆)alkyle éventuellement substitué par un ou plusieurs radicaux halo identiques ou différents, (C₁-C₆)alkyl-thio, (C₁-C₆)alkyl-sulfonyle, (C₁-C₆)alkoxy éventuellement substitué par un ou plusieurs radicaux halo identiques ou différents, aryl-alkoxy, (C₁-C₆)alkoxy-carbonyle, -(CH₂)ₙ-NR₃R₄ et -NH-C(O)-CH(R_{A})-NR₅R₆ ;
      R₃ et R₄ représentent, indépendamment, H, (C₁-C₆)alkyle ou (C₁-C₆)alkyl-carbonyle ;
      R₅ et R₆ représentent, indépendamment, H ou (C₁-C₆)alkyle ;
      R_{A} représente le résidu associé à l'acide aminé de formule NH₂-CH(R_{A})-C(O)-OH ;
      R_{N} représente l'hydrogène ou un radical (C₁-C₆)alkyle ; ou un sel pharmaceutiquement acceptable de ce dernier.

De préférence également, l'invention concerne un composé de formule (I) tel que défini ci-dessus et caractérisé en ce que Z représente un ou plusieurs substituants, identiques ou différents, de formule -Z₂-Z'₂, et très préférentiellement Z est en position méta et/ou para ; ou un sel pharmaceutiquement acceptable de ce dernier.

L'invention concerne très préférentiellement un composé de formule (I) tel que défini ci-dessus et caractérisé en ce que Z₂ représente-O-, -S-, -SO₂- ou -C(O)-, et plus particulièrement -O-; ou un sel pharmaceutiquement acceptable de ce dernier.

L'invention concerne très préférentiellement un composé de formule (I) tel que défini ci-dessus et caractérisé en ce que Z₂ représente préférentiellement également -NR_{N}- ; ou un sel pharmaceutiquement acceptable de ce dernier.

Préférentiellement, Z'₂ représente phényle ou phényle substitué par un ou plusieurs radicaux identiques ou différents choisis parmi :
halo, nitro, cyano, hydroxy, (C₁-C₆)alkyle éventuellement substitué par un ou plusieurs radicaux halo identiques ou différents, (C₁-C₆)alkyl-thio, (C₁-C₆)alkyl-sulfonyle, (C₁-C₆)alkoxy éventuellement substitué par un ou plusieurs radicaux halo identiques ou différents, benzyloxy, (C₁-C₆)alkoxy-carbonyle, phosphate, -(CH₂)ₙ-NR₃R₄ et NH-C(O)-CH(R_{A})-NR₅R₆ ;
   R₃ et R₄ représentent, indépendamment, H, (C₁-C₆)alkyle, (C₁-C₆)alkyl-carbonyle ou (C₁-C₆)alkyl-sulfonyle ;
   R_{N} représente l'hydrogène ou un radical (C₁-C₆)alkyle;
   R₅ et R₆ représentent, indépendamment, H ou (C₁-C₆)alkyle ; et
   R_{A} représente le résidu associé à l'acide aminé de formule NH₂-CH(R_{A})-C(O)-OH, et plus particulièrement
      Z'₂ représente phényle substitué par un ou plusieurs radicaux identiques ou différents choisis parmi : halo, nitro, cyano, hydroxy, (C₁-C₆)alkyl-sulfonyle, (C₁-C₆)alkoxy, -(CH₂)ₙ-NR₃R₄ et -NH-C(O)-CH(R_{A})-NR₅R₆ ;
      R₃ et R₄ représentent, indépendamment, H, (C₁-C₆)alkyle ou (C₁-C₆)alkyl-carbonyle ;
      R₅ et R₆ représentent, indépendamment, H ou (C₁-C₆)alkyle ; ou un sel pharmaceutiquement acceptable de ce dernier.

L'invention concerne très préférentiellement également un composé de formule (I) tel que défini ci-dessus et caractérisé en ce que Z'₂ représente phényle substitué par au moins deux radicaux identiques ou différents choisis parmi : fluoro, nitro, cyano, hydroxy, (C₁-C₆)alkyl-sulfonyle, (C₁-C₆)alkoxy, -NH₂ et -NH-C(O)-CH(R_{A})-NR₅R₆ ;

R₅ et R₆ représentent, indépendamment, H ou (C₁-C₆)alkyle; ou un sel pharmaceutiquement acceptable de ce dernier.

Préférentiellement également, l'invention concerne un composé de formule (I) tel que défini ci-dessus et caractérisé en ce que
Z'₂ représente le radical pyridinyle, pyrimidinyle, pyrazinyle, triazolyle, furyle, thiényle, purinyle, triazinyle, pyrrazolo-pyrimidinyle, quinoxalinyle ou indolyle,
chacun de ces radicaux étant éventuellement substitué par un ou plusieurs radicaux identiques ou différents choisis parmi : halo, nitro, cyano, hydroxy, (C₁-C₆)alkyle et -NH₂ ; ou un sel pharmaceutiquement acceptable de ce dernier.

L'invention a également pour objet des composés tels qu'illustrés dans la partie expérimentale et caractérisés en ce qu'ils répondent à l'une des formules suivantes:
4-[4-(4-fluorophénoxy)phényl]-2-(phénoxyméthyl)-1H-imidazole ;
4-(1,1'-biphényl-4-yl)-2-[(phénylthio)méthyl]-1H-imidazole ;
4-(1,1'-biphényl-4-yl)-2-(phénoxyméthyl)-1H-imidazole;
4-[4-(4-fluorophénoxy)phényl]-2-[(phénylthio)méthyl]-1H-imidazole;
2-[(4-fluorophénoxy)méthyl]-4-[4-(4-fluorophénoxy)phényl]-1H-imidazole;
2-(phénoxyméthyl)-4-[4-(phénylthio)phényl]-1H-imidazole;
2-(phénoxyméthyl)-4-[4-(phénylsulfonyl)phényl]-1H-imidazole ;
4-{4-[(2-fluorobenzyl)oxy]phényl}-2-(phénoxyméthyl)-1H-imidazole;
trifluoroacétate de 2-(phénoxyméthyl)-4-(4-phénoxyphényl)-1H-imidazole;
trifluoroacétate de 4-[4-(4-bromophénoxy)phényl]-2-(phénoxyméthyl)-1H-imidazole;
4-[4-(1H-imidazol-1-yl)phényl]-2-(phénoxyméthyl)-1H-imidazole ;
4-[4-(4-méthoxyphénoxy)phényl]-2-(phénoxyméthyl)-1H-imidazole ;
4-(4-hexylphényl)-2-(phénoxyméthyl)-1H-imidazole
4-(4-butoxyphényl)-2-(phénoxyméthyl)-1H-imidazole;
4-[4-(4-nitrophénoxy)phényl]-2-(phénoxyméthyl)-1H-imidazole;
4-(2-{4-[2-(phénoxyméthyl)-1H-imidazol-4-yl]phénoxy}éthyl) morpholine ;
chlorhydrate de 1-(2-{4-[2-(phénoxyméthyl)-1H-imidazol-4-yl]phénoxy} éthyl) pipéridine;
chlorhydrate de N,N-diméthyl-N-(2-{4-[2-(phénoxyméthyl)-1H-imidazol-4-yl] phénoxy}éthyl) amine;
4-[4-(2-méthoxyéthoxy)phényl]-2-(phénoxyméthyl)-1H-imidazole ;
2-(phénoxyméthyl)-4-[4-(4,4,4-trifluorobutoxy)phényl]-1H-imidazole;
4-[4-(4-fluorophénoxy)phényl]-5-méthyl-2-(phénoxyméthyl)-1H-imidazole ;
4-fluoro-N-{4-[2-(phénoxyméthyl)-1H-imidazol-4-yl]phényl} benzamide ;
4-{4-[2-(phénoxyrnéthyl)-1H-imidazol-4-yl]phénoxy}benzonitrile;
4-[2-(phénoxyméthyl)-1H-imidazol-4-yl] benzoate d'éthyle ;
4-{4-[2-(phénoxyméthyl)-1H-imidazol-4-yl]phénoxy}benzoate d'éthyle ;
4-{4-[4-(méthylthio)phénoxy]phényl}-2-(phénoxyméthyl)-1H-imidazole ;
4-{4-[4-(méthylsulfonyl)phénoxy]phényl}-2-(phénoxyméthyl)-1H-imidazole;
chlorhydrate de 4-{4-[2-(phénoxyméthyl)-1H-imidazol-4-yl]phénoxy}aniline ;
trifluoroacétate de {4-[2-(phénoxyméthyl)-1H-imidazol-4-yl]phényl}phényl méthanone ;
trifluoroacétate de N-(4-fluorophényl)-4-[2-(phénoxyméthyl)-1H-imidazol-4-yl] benzamide ;
4-[4-(3-nitrophénoxy)phényl]-2-(phénoxyméthyl)-1H-imidazole ;
chlorhydrate de 3-{4-[2-(phénoxyméthyl)-1H-imidazol-4-yl]phénoxy} aniline ;
4-{4-[4-(benzyloxy)phénoxy]phényl}-2-(phénoxyméthyl)-1H-imidazole ;
4-{4-[2-(phénoxyméthyl)-1H-imidazol-4-yl]phénoxy}phénol ;
4-[4-(3-fluorophénoxy)phényl]-2-(phénoxymethyl)-1H-imidazole ;
N-(4- {4-[2-(phénoxyméthyl)-1H-imidazol-4-yl]phénoxy}phényl) acétamide ;
trifluoroacétate de 2-nitro-4-{4-[2-(phénoxyméthyl)-1H-imidazol-4-yl]phénoxy} aniline ;
N-méthyl-N-(4-{4-[2-(phénoxyméthyl)-1H-imidazol-4-yl]phénoxy}phényl) amine ;
3-{4-[2-(phénoxyméthyl)-1H-imidazol-4-yl]phénoxy}benzonitrile ;
4-[4-(2-nitrophénoxy)phényl]-2-(phénoxyméthyl)-1H-imidazole ;
chlorhydrate de 2-{4-[2-(phénoxyméthyl)-1H-imidazol-4-yl]phénoxy}aniline ;
chlorhydrate de 1-(4-{4-[2-(phénoxyméthyl)-1H-imidazol-4-yl]phénoxy}phényl) méthanamine ;
chlorhydrate de 1-(3-{4-[2-(phénoxyméthyl)-1H-imidazol-4-yl]phénoxy}phényl) méthanamine ;
4-[4-(3-bromophénoxy)phényl]-2-(phénoxyméthyl)-1H-imidazole ;
chlorhydrate de 2-fluoro-4-{4-[2-(phénoxyméthyl)-1H-imidazol-4-yl]phénoxy} aniline ;
4-[4-(3-chlorophénoxy)phényl]-2-(phénoxyméthyl)-1H-imidazole;
4-[4-(3,5-difluorophénoxy)phényl]-2-(phénoxyméthyl)-1H-imidazole ;
4-(4-benzylphényl)-2-(phénoxyméthyl)-1H-imidazole ;
4-[4-(3-méthylphénoxy)phényl]-2-(phénoxyméthyl)-1H-imidazole ;
chlorhydrate de 4-[4-(2-chlorophénoxy)phényl]-2-(phénoxyméthyl)-1H-imidazole ;
4-[4-(2-fluorophénoxy)phényl]-2-(phénoxyméthyl)-1H-imidazole ;
4-[4-(3,4-difluorophénoxy)phényl]-2-(phénoxyméthyl)-1H-imidazole ;
chlorhydrate de N¹-(4-{4-[2-(phénoxyméthyl)-1H-imidazol-4-yl]phénoxy}phényl) glycinamide;
4-[4-(2,5-difluorophénoxy)phényl]-2-(phénoxyméthyl)-1H-imidazole ;
4-[4-(2,4-difluorophénoxy)phényl]-2-(phénoxyméthyl)-1H-imidazole ;
4-[4-(2,3-difluorophénoxy)phényl]-2-(phénoxyméthyl)-1H-imidazole ;
4-[4-(2,6-difluorophénoxy)phényl]-2-(phénoxyméthyl)-1H-imidazole.

L'invention a pour objet également un procédé de préparation d'un composé selon l'invention et caractérisé en ce que l'on fait réagir un composé de formule 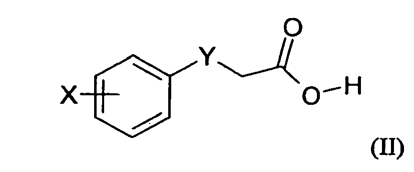 dans laquelle X et Y ont la signification indiquée ci-dessus, avec une base pour former le composé (II) sous une forme salifiée, puis avec l'α-halogéno-cétone de formule 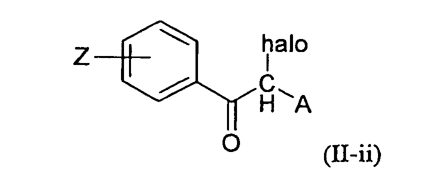 dans laquelle Z et A ont la signification indiquée ci-dessus, dans un solvant inerte,
puis le céto-ester ainsi obtenu est cyclisé en présence d'un sel d'ammonium pour donner le composé de formule (I).

L'acide de formule générale (II) est traité avec une base telle que Cs₂CO₃ dans un solvant tel que le méthanol ou l'éthanol. Au sel de césium récupéré est ajoutée l'α-halogéno-cétone de formule générale (II-ii) dans un solvant inerte tel que le diméthylformamide. Le cétoester intermédiaire conduit, par chauffage à reflux dans un solvant apolaire aprotique tel que le xylène (mélange d'isomères) ou le toluène, en présence d'un large excès de sel d'ammonium tel que l'acétate d'ammonium (15 ou 20 équivalents par exemple), au dérivé imidazole de formule générale (I) (l'eau formée étant éliminée en cours de réaction).

L'α-halogéno-cétone de formule générale (II-ii) peut être préparée à partir du dérivé cétonique suivant : 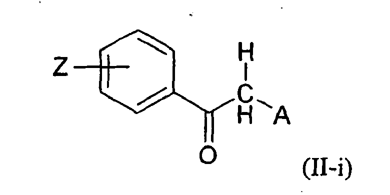 dans lequel Z et A sont tels que définis ci-dessus.

Le dérivé cétonique de formule générale (II-i) est converti en l'α-halogéno-cétone correspondante de formule générale (II-ii). De préférence, le dérivé cétonique de formule générale (II-i) est converti en l'α-bromo-cétone, par réaction avec un agent de bromation tel que CuBr₂ (J. Org.Chem. (1964), 29, 3459), du brome dans de l'éthanol ou de l'acide acétique (J. Het. Chem. (1988), 25, 337 ; J. Med. Chem. (1988), 31(10), 1910-1918), du N-bromosuccinimide (J. Amer. Chem. Soc. (1980), 102, 2838) en présence d'acide acétique dans un solvant tel que l'acétate d'éthyle ou le dichlorométhane; HBr dans de l'éther (Biorg. Med. Chem. Lett. (1996), 6(3), 253-258 ; J. Am. Chem. Soc.(1999), 121, 24) ou encore à l'aide d'une résine de bromation (J. Macromol. Sci. Chem. (1977), A11, (3) 507-514).

Les composés (II-i) peuvent être préparés selon les procédures connues de l'homme de l'art (Schmid, C. R. ; Sluka, J. P. ; Duke, K. M. Tetrahedron Lett. 1999, 40, 675-678 ; Hogenkamp, D. J.; Upasani, R.; Nguyen, P.; WO 00/57877. Chem. Abstr. 2000, 133, 28179).

Les composés de formule générale (I) peuvent être également préparés en condensant au reflux dans un solvant inerte polaire comme le diméthylformamide, un composé de départ de formule (II-iii) dans laquelle X et Y ont la signification indiquée ci-dessus et l'α-halogéno-cétone de formule générale (II-ii) dans lequel Z et A sont tels que définis ci-dessus.

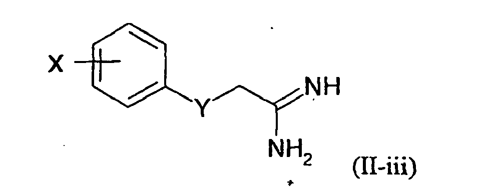

L'invention a donc pour objet également un procédé de préparation d'un composé selon l'invention et caractérisé en ce que l'on condense au reflux dans un solvant inerte polaire, un composé de formule (II-iii) 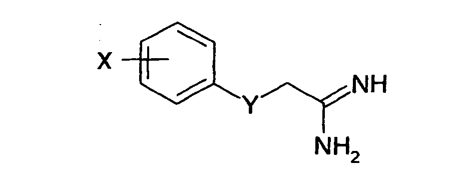 dans laquelle X et Y ont la signification indiquée ci-dessus, et l'α-halogéno-cétone de formule générale (II-ii) dans lequel Z et A sont tels que définis ci-dessus.

Les composés de formule (I) de la présente invention possèdent d'intéressantes propriétés pharmacologiques. C'est ainsi que l'on a découvert que les composés de formule (I) de la présente invention possèdent une activité anti-tumorale, et plus particulièrement une activité inhibitrice de la polymérisation de la tubuline.

Les composés de la présente invention peuvent ainsi être utilisés dans différentes applications thérapeutiques. Ils peuvent avantageusement être utilisés pour le traitement de tumeurs ou de cancers tels que définis précédemment et de préférence les cancers du colon, de la prostate, du pancréas et les mélanomes. On trouvera ci-après, dans la partie expérimentale, une illustration des propriétés pharmacologiques des composés de l'invention.

La présente demande a également pour objet, des compositions pharmaceutiques contenant, à titre de principe actif, au moins un composé de formule (I) tel que défini ci-dessus, ainsi que les sels d'addition avec les acides minéraux ou organiques pharmaceutiquement acceptables dudit composé de formule I, en association avec un support pharmaceutiquement acceptable.

La présente demande a également pour objet l'utilisation d'un composé de formule (I) selon la présente invention, pour la préparation d'un médicament anti-tumoral.

La présente demande a également pour objet l'utilisation d'un composé de formule (I) selon la présente invention, pour la préparation d'un médicament destiné à inhiber la polymérisation de la tubuline.

Des dérivés d'imidazole ont été décrits dans la demande WO 01/44201 comme antagonistes des récepteurs Y5.

La présente demande a donc également pour objet, l'utilisation d'un composé de formule (I') 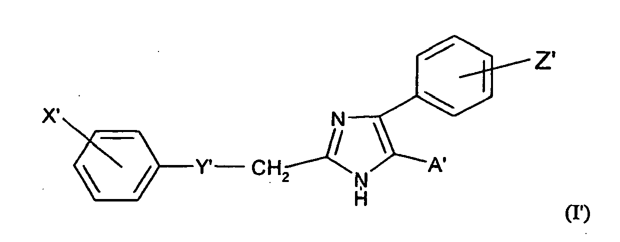 sous forme racémique, d'énantiomère ou toutes combinaisons de ces formes, et dans laquelle
X' représente un ou plusieurs substituants identiques ou différents choisis parmi H et halo ;
Y' représente -O- ou -S- ;
A' représente H ou (C₁-C₆)alkyle ;
Z' représente un ou plusieurs substituants identiques ou différents choisis parmi :
   - (C₁-C₆)alkyle éventuellement substitué par un ou plusieurs radicaux halo identiques ou différents ;
   - aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, -(CH₂)ₙ-NR₃R₄, (C₁-C₆)alkyl-sulfonyle, (C₁-C₆)alkyl-thio, (C₁-C₆)alkoxy éventuellement substitué par un ou plusieurs radicaux halo identiques ou différents, (C₁-C₆)alkoxy-carbonyle, phosphate, sulfate, glycoside et NH-C(O)-CH(R_{A})-NR₅R₆ ;
   - aryl-(C₁-C₆)alkyle ;
   - hétéroaryle ;
   - -Z₁-Z'₁;
   - -NR_{N}-C(O)-Z'₂ ; ou
   - -Z₂-Z'₂ ;
Z₁ représente -O- ,-C(O)-O-, -NR_{N}-C(O)- ou -C(O)-NR_{N}- ;
Z'₁ représente un radical (C₁-C₁₀)alkyle ; aryle-(C₁-C₆)alkyle dont le radical aryle est éventuellement substitué par un ou plusieurs halo identiques ou différents ; ou (C₁-C₆)alkyle substitué par un ou plusieurs substituants choisis parmi : halo, (C₁-C₆)alkoxy, (C₁-C₆)alkylthio et -NR₁R₂;
R₁ et R₂ représentent, indépendamment, H ou (C₁-C₆)alkyle, ou bien forment ensemble avec l'atome d'azote sur lequel ils sont rattachés, un hétérocycloalkyle éventuellement substitué par (C₁-C₆)alkyle ;
Z₂ représente -O-, -S-, -SO₂-, -C(O)-, -C(O)-NR_{N}- ou -NR_{N}- ;
Z'₂ représente un radical aryle ou hétéroaryle, les radicaux aryle et hétéroaryle étant éventuellement substitués par un ou plusieurs radicaux identiques ou différents choisis parmi :
   halo, nitro, cyano, hydroxy, (C₁-C₆)alkyle éventuellement substitué par un ou plusieurs radicaux halo identiques ou différents, (C₁-C₆)alkyl-thio, (C₁-C₆)alkyl-sulfonyle, (C₁-C₆)alkoxy éventuellement substitué par un ou plusieurs radicaux halo identiques ou différents, aryl-alkoxy, (C₁-C₆)alkoxy-carbonyle, phosphate, sulfate, glycoside, -(CH₂)ₙ-NR₃R₄ et NH-C(O)-CH(R_{A})-NR₅R₆ ;
R₃ et R₄ représentent, indépendamment, H, (C₁-C₆)alkyle, (C₁-C₆)alkyl-carbonyle ou (C₁-C₆)alkyl-sulfonyle, ou bien R₃ et R₄ forment ensemble avec l'atome d'azote sur lequel ils sont rattachés, un hétéroaryle ou un hétérocycloalkyle éventuellement substitué par (C₁-C₆)alkyle ;
R₅ et R₆ représentent, indépendamment, H ou (C₁-C₆)alkyle ;
R_{A} représente le résidu associé à l'acide aminé de formule NH₂-CH(R_{A})-C(O)-OH ;
R_{N} représente l'hydrogène ou un radical (C₁-C₆)alkyle ;
n représente un entier de 0 à 3 ; ou un sel pharmaceutiquement acceptable de ces composés, pour la préparation d'un médicament anti-tumoral.

La présente demande a également pour objet l'utilisation des composés de formule (I') tels que définis ci-dessus, pour la préparation d'un médicament destiné à inhiber la polymérisation de la tubuline.

Les composés de la présente invention peuvent être administrés seuls ou en association avec d'autres agents à activité anti-tumorale. Parmi les agents à activité anti-tumorale, on peut citer : les inhibiteurs de topoisomérase I tels que le diflomotécan, l'irinotécan ou le topotécan ; les inhibiteurs de topoisomèrase II ; les agents alkylants tels que la cyclophosphamide, les fosfamides ou le melphalan ; les dérivés du platine tels que cisplatine, carboplatine ou oxaliplatine ; les agents antibiotiques tels que la bléomycine ou la mitomycine ; les antimétabolites tels que le 5-fluorouracil; et les agents hormonaux.

L'administration d'une composition selon l'invention peut également être associée à de la radiothérapie.

La composition pharmaceutique peut être sous forme d'un solide, par exemple, des poudres, des granules, des comprimés, des gélules. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par voie intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

Les exemples sont présentés pour illustrer les procédures ci-dessus et ne doivent en aucun cas être considérés comme une limite à la portée de l'invention.

### Partie expérimentale :

### Exemple 1 : 4-[4-(4-fluorophénoxy)phényl]-2-(phénoxyméthyl)-1H-imidazole

### 1.1) 1-[4-(4-fluorophénoxy)phényl]éthanone

17,7 ml de 4-fluoroacétophénone (0,145 mol), 17,84 g de 4-fluorophénol (0,16 mol) et du carbonate de potassium (50 g, 0,36 mol) dans 220 ml de diméthylformamide anhydre sont chauffés au reflux pendant 4 heures.

Le mélange est refroidi puis on ajoute 200 ml d'acétate d'éthyle et 200 ml d'eau. Après décantation, la phase organique est récupérée, lavée avec une solution de soude 2N puis avec une solution saturée en chlorure de sodium. La phase organique est alors séchée sur Na₂SO₄ et le solvant évaporé. Le résidu est alors retraité pendant 30 min sous agitation dans 50 ml d'isopentane puis filtré sur fritté. On obtient une poudre de couleur beige.

RMN ¹H (δ ppm, DMSO): 2,53 (s, 3H) ; 7,01-7,03 (d, 2H); 7,16-7,31 (m, 4H) ; 7,96-7,99 (d, 2H)

Point de fusion : 70° C

### 1.2) 2-bromo-1-[4-(4-fluorophénoxy)phényl]éthanone

Une solution de 1-[4-(4-fluorophénoxy)phényl]éthanone (17,7 g, 0,077 mol) dans 220 ml d'éthanol est refroidie aux environs de 0° C. Le brome (4,8 ml, 0,096 mol) est ajouté goutte à goutte à la seringue. On laisse la température revenir à la température ambiante et on agite pendant 2 heures. Après évaporation du solvant puis agitation pendant 10 heures dans de l'isopentane, le résidu est filtré sur fritté et séché sous cloche à vide. On obtient une poudre de couleur beige.

RMN ¹H (δ ppm, DMSO): 4,85 (s, 2H); 7,03-7,05 (d, 2H); 7,18-7,32 (m, 4H); 8,00-8,02 (d, 2H)
Point de fusion : 56° C

### 1.3) 4-[4-(4-fluorophénoxy)phényl]-2-(phénoxyméthyl)-1H-imidazole

Un mélange contenant de l'acide phénoxyacétique (0,5 g, 3,3 mmol) et du carbonate de césium (0,53 g, 1,65 mmol) dans 10 ml de méthanol anhydre est agité pendant une heure. Ce mélange est évaporé à sec puis dilué avec 20 ml de diméthylformamide. 1 g de 2-bromo-1-[4-(4-fluorophénoxy)phényl]éthanone (3,3 mmol) est ajouté puis le mélange résultant est agité durant 2 heures. Le solvant est évaporé à l'aide d'une pompe à membranes. 30 ml d'acétate d'éthyle sont ajoutés et le bromure de césium est filtré sur fritté. Après évaporation du solvant, le résidu est dilué avec 50 ml de xylène puis on ajoute de l'acétate d'ammonium (3,8 g, 0,066 mol) et le mélange maintenu par un Dean Stark est chauffé au reflux pendant 2 heures. Puis on verse dans de l'eau glacée à laquelle on ajoute 50 ml d'acétate d'éthyle. Après décantation, la phase organique est lavée avec une solution saturée en chlorure de sodium. La phase organique est ensuite séchée sur sulfate de magnésium et le solvant évaporé. L'huile obtenue cristallise dans l'éther isopropylique et quelques gouttes d'éthanol. On laisse l'agitation puis on filtre sur fritté en rinçant à l'éther isopropylique et à l'isopentane avant de le sécher sous vide. Le solide obtenu est purifié par chromatographie sur colonne de silice (éluant : acétate d'éthyle-heptane : 1-3). Après évaporation du solvant, le solide est lavé dans l'éther isopropylique puis filtré sur fritté. On obtient une poudre de couleur beige.

RMN ¹H (δ ppm, DMSO) : 5, 08 (s, 2H) ; 6,94-7,79 (m, 14H) ; 12,38-12,72 (s large, 1H)
MH+ expérimental = 361,1 ; MH+ théorique = 360,39
% C 73,32 ; % H 4,75 ; % N 7,77 (théorique) ; % C 73,17 ; % H 4,78 ; % N 7,63 (mesuré)
Point de fusion : 188-190° C

### Exemple 2 : 4-(1,1'-biphényl-4-yl)-2-[(phénylthio)méthyl]-1H-imidazole

Ce composé est synthétisé selon une méthode analogue à celle décrite à l'exemple 1.

MH+ expérimental = 343,10 ; MH+ théorique = 342,46
Point de fusion : 150-152° C

### Exemple 3 : 4-(1,1'-biphényl-4-yl)-2-(phénoxyméthyl)-1H-imidazole

Ce composé est synthétisé selon une méthode analogue à celle décrite à l'exemple 1.

MH+ expérimental = 327,20 ; MH+ théorique = 326,40
Point de fusion : 185-187° C

### Exemple 4 : 4-[4-(4-fluorophénoxy)phényl]-2-[(phénylthio)méthyl]-1H-imidazole

Ce composé est synthétisé selon une méthode analogue à celle décrite à l'exemple 1.

MH+ expérimental = 377,10 ; MH+ théorique = 376,45
Point de fusion : 108-110° C

### Exemple 5 : 2-[(4-fluorophénoxy)méthyl]-4-[4-(4-fluorophénoxy)phényl]-1H-imidazole

Ce composé est synthétisé selon une méthode analogue à celle décrite à l'exemple 1.
MH+ expérimental = 379,00 ; MH+ théorique = 378,38
Point de fusion : 193-195° C

### Exemple 6 : 2-(phénoxyméthyl)-4-[4-(phénylthio)phényl]-1H-imidazole

Ce composé est synthétisé selon une méthode analogue à celle décrite à l'exemple 1.
MH+ expérimental = 359,10 ; MH+ théorique = 358,46
Point de fusion : 144-146° C

### Exemple 7 : 2-(phénoxyméthyl)-4-[4-(phénylsulfonyl)phényl]-1H-imidazole

Une solution d'eau oxygénée (1,3 ml d'une solution à 30 % dans l'eau) est ajoutée à 0,133 g (0,00037 mol) de 2-(phénoxyméthyl)-4-[4-(phénylthio)phényl]-IH-imidazole dissous dans 1 ml d'acide acétique. On agite pendant environ 20 h puis on évapore à sec. 20 ml d'eau et 30 ml d'acétate d'éthyle sont alors rajoutés. La phase organique est extraite puis séchée sur sulfate de sodium. Après évaporation du solvant, le résidu obtenu est traité avec un mélange de solvants tel que isopentane - éther diéthylique en proportion 1-1. Après filtration sur fritté, le solide obtenu est lavé à l'éther diéthylique puis séché pour obtenir une poudre de couleur jaune pâle.
RMN ¹H (δ ppm, DMSO) : 5,09 (s, 2H) ; 6,95-8,02 (m, 15H) ; 12,38-12,72 (s large, 1H)
MH+ expérimental = 391,20 ; MH+ théorique = 390,46
Point de fusion : 192-194° C

### Exemple 8 : 4-{4-[(2-fluorobenzyl)oxy]phényl}-2-(phénoxyméthyl)-1H-imidazole

Ce composé est synthétisé selon une méthode analogue à celle décrite à l'exemple 1.
MH+ expérimental = 375,00 ; MH+ théorique =374,41
Point de fusion : 182-183° C

### Exemple 9 : trifluoroacétate de 2-(phénoxyméthyl)-4-(4-phénoxyphényl)-1H-imidazole

Ce composé est synthétisé selon une méthode analogue à celle décrite à l'exemple 1.
MH+ expérimental = 343,20 ; MH+ théorique = 342,40
Point de fusion : 112-114° C

### Exemple 10 : trifluoroacétate de 4-[4-(4-bromophénoxy)phényl]-2-(phénoxyméthyl)-1H-imidazole

Ce composé est synthétisé selon une méthode analogue à celle décrite à l'exemple 1.
MH+ expérimental = 421,10 ; MH+ théorique = 421,29
Point de fusion : 174-176° C

### Exemple 11 : 4-[4-(1H-imidazol-1-yl)phényl]-2-(phénoxyméthyl)-1H-imidazole

Ce composé est synthétisé selon une méthode analogue à celle décrite à l'exemple 1.
MH+ expérimental = 317,20 ; MH+ théorique = 316,36
Point de fusion : 194-196° C

### Exemple 12 : 4-[4-(4-méthoxyphénoxy)phényl]-2-(phénoxyméthyl)-1H-imidazole

Ce composé est synthétisé selon une méthode analogue à celle décrite à l'exemple 1.
MH+ expérimental = 373,20 ; MH+ théorique =373,42
Point de fusion : 132-134° C

### Exemple 13 : 4-(4-hexylphényl)-2-(phénoxyméthyl)-1H-imidazole

Ce composé est synthétisé selon une méthode analogue à celle décrite à l'exemple 1.
MH+ expérimental = 335,20 ; MH+ théorique = 334,46
Point de fusion: 151-153° C

### Exemple 14 : 4-(4-butoxyphényl)-2-(phénoxyméthyl)-1H-imidazole

Ce composé est synthétisé selon une méthode analogue à celle décrite à l'exemple 1.
MH+ expérimental = 323,20 ; MH+ théorique = 322,41
Point de fusion : 179-181° C

### Exemple 15 : 4-[4-(4-nitrophénoxy)phényl]-2-(phénoxyméthyl)-1H-imidazole

Ce composé est synthétisé selon une méthode analogue à celle décrite à l'exemple 1.
MH+ expérimental = 388,20 ; MH+ théorique = 387,39
Point de fusion : 187-189° C

### Exemple 16 : 4-(2-{4-[2-(phénoxyméthyl)-1H-imidazol-4-yl]phénoxy}éthyl) morpholine

### 16.1) 1-[4-(2-morpholin-4-yl éthoxy)phényl]éthanone

De l'hydrure de sodium (3,18 g ; 0,0795 mol d'une poudre dispersée à 60 %) est ajouté à 23° C à une solution contenant de la 4-(2-hydroxyéthyl)morpholine (9,40 g, 0,072 mol) dans du diméthylformamide (60 ml). L'agitation est maintenue pendant 30 minutes puis le composé 4-fluoroacétophénone (5g, 0,0362 mol) est ajouté. On laisse agiter une heure à 23° C puis on refroidit à 0° C et de l'eau est rajoutée. Après ajout d'acétate d'éthyle puis extraction, la phase organique est lavée avec une solution saturée en chlorure de sodium, séchée sur sulfate de sodium puis le solvant est évaporé. Le résidu obtenu est adsorbé sur silice puis purifié par chromatographie sur colonne de silice de type Biotage (éluant : acétate d'éthyle-heptane : 6-1). On obtient une huile de couleur orange.
RMN ¹H (δ ppm, DMSO) : 2,34-2,54 (m, 7H) ; 2,66-2,71 (m, 2H) ; 3,57-3,71 (m, 4H) ; 4,16-4,19 (m, 2H) ; 6,97-7,07(d, 2H) ; 7,84-7,92 (d, 2H)

### 16.2) chlorhydrate de 2-bromo-1-[4-(2-morpholin-4-yléthoxy)phényl]éthanone

1,02 ml de brome (0,0205 mol) est ajouté goutte à goutte, sous argon, à une solution refroidie à 0° C de 1-[4-(2-morpholin-4-yléthoxy)phényl]éthanone (4,09 g ; 0,0164 mol) dans de l'éthanol (65 ml). On laisse ensuite l'agitation 30 minutes à 23° C puis le solvant et les traces de brome sont évaporés à l'évaporateur rotatif sous vide. Le résidu est ensuite agité dans l'éther diéthylique avec quelques gouttes d'éthanol. Le solide obtenu est filtré puis séché. On obtient une poudre de couleur beige.
RMN ¹H (δ ppm, DMSO) : 2,34-2,54 (m, 3H) ; 2,66-2,71 (m, 2H) ; 3,57-3,71 (m, 4H) ; 4,16-4,19 (m, 2H) ; 4,84 (s, 2H) ; 6,97-7,07(d, 2H) ; 7,84-7,92 (d, 2H) ; 10 (sé, 1H)
16.3) 4-(2- {4-[2-(phénoxyméthyl)-1H-imidazol-4-yl]phénoxy}éthyl)morpholine

Le mode opératoire est analogue à celui décrit à l'exemple 1.3 en utilisant comme produit de départ le chlorhydrate de 2-bromo-1-[4-(2-morpholin-4-yléthoxy)phényl]éthanone décrit ci-dessus et en utilisant un équivalent de carbonate de césium.
RMN ¹H (δ ppm, DMSO) : 2,46-2,50 (m, 4H) ; 2,66-2,69 (m, 2H) ; 3,56-3,58 (m, 4H); 4,06-4,09 (m, 2H) ; 5,04 (s, 2H) ; 6,90-7,68 (m, 10H); 12,50 (s large, 1H)
MH+ expérimental = 380,20 ; MH+ théorique = 379,46.
Point de fusion : 144-146° C

### Exemple 17 : chlorhydrate de 1-(2-{4-[2-(phénoxyméthyl)-1H-imidazol-4-yl] phénoxy}éthyl)pipéridine

Ce composé est synthétisé selon une méthode analogue à celle décrite à l'exemple 16.
MH+ expérimental = 378,30 ; MH+ théorique = 377,48
Point de fusion : 227-229° C

### Exemple 18 : chlorhydrate de N,N-diméthyl-N-(2-{4-[2-(phénoxyméthyl)-1H-imidazol-4-yl]phénoxy}éthyl)amine

Ce composé est synthétisé selon une méthode analogue à celle décrite à l'exemple 16.
MH+ expérimental = 338,30 ; MH+ théorique = 337,42
Point de fusion : 206-208° C

### Exemple 19 : 4-[4-(2-méthoxyéthoxy)phényl]-2-(phénoxyméthyl)-1H-imidazole

Ce composé est synthétisé selon une méthode analogue à celle décrite à l'exemple 16.
MH+ expérimental = 325,20 ; MH+ théorique = 324,38
Point de fusion : 159-161°C

### Exemple 20 : 2-(phénoxyméthyl)-4-[4-(4,4,4-trifluorobutoxy)phényl]-1H-imidazole

Ce composé est synthétisé selon une méthode analogue à celle décrite à l'exemple 16.
MH+ expérimental = 377,20 ; MH+ théorique = 376,38
Point de fusion : 194-196° C

### Exemple 21 : 4-[4-(4-fluorophénoxy)phényl]-5-méthyl-2-(phénoxyméthyl)-1H-imidazole

### 21.1) 1-[4-(4-fluorophénoxy)phényl]propan-1-one

Ce composé est synthétisé selon une méthode analogue à celle décrite à l'exemple 1.1.

### 21.2) 2-bromo-1-[4-(4-fluorophénoxy)phényl]propan-1-one

Ce composé est synthétisé selon une méthode analogue à celle décrite à l'exemple 1.2.

### 21.3) 4-[4-(4-fluorophénoxy)phényl]-5-méthyl-2-(phénoxyméthyl)-1H-imidazole

620 mg de chlorhydrate de 2-phénoxyéthanimidamide (0,00333 mol) sont désalifiés dans du dichlorométhane par action d'une solution d'hydroxyde de sodium 3N. Après décantation et extraction de la phase aqueuse par du dichlorométhane, la phase organique est séchée sur sulfate de sodium puis évaporée à sec. La poudre blanche obtenue est solubilisée dans du diméthylformamide (30 ml). 275 mg de 2-bromo-1-[4-(4-fluorophénoxy)phényl]propan-1-one (0,000851 mol) sont ajoutés. Le mélange est chauffé à 50° C pendant 20 h puis on revient à 23° C avant d'ajouter 25 ml d'eau et 30 ml d'acétate d'éthyle. Après extraction avec de l'acétate d'éthyle, la phase organique est séchée sur sulfate de sodium puis évaporée à l'évaporateur rotatif sous vide. Le résidu obtenu est adsorbé sur silice puis purifié par chromatographie sur colonne de silice de type Biotage (éluant : acétate d'éthyle-heptane : 2-8). On obtient une mousse de couleur jaune pâle.
RMN ¹H (δ ppm, DMSO) : 2,37 (s, 3H); 5,00 (s, 2H); 6,93-7,65 (m, 13H); 12,19 (s large, 1H)
MH+ expérimental = 375,20; MH+ théorique = 374,41
Point de fusion : <40° C

### Exemple 22 : 4-fluoro-N-{4-[2-(phénoxyméthyl)-1H-imidazol-4-yl]phényl} benzamide

### 22.1) 4-(4-azidophényl)-2-(phénoxyméthyl)-1H-imidazole

Ce composé est préparé d'une façon analogue à la méthode décrite pour l'exemple 1.3 en utilisant comme produits de départ l'acide phénoxyacétique (2 g ; 0,01314 mol) et le bromure de 4-azidophénacyle (3,15 g, 0,01314 mol). On obtient une poudre de couleur jaune.
MH+ expérimental = 292,20 ; MH+ théorique = 291,31.

### 22.2) 4-[2-(phénoxyméthyl)-1H-imidazol-4-yl]aniline

Dans un réacteur de 100 ml, 549 mg de 4-(4-azidophényl)-2-(phénoxyméthyl)-1H-imidazole (0,00188 mol) sont hydrogénés pendant 18 heures sous une pression d'hydrogène de 2,5 bars avec une quantité catalytique de palladium adsorbé sur charbon (10 % massique). Après filtration sur filtre millipore puis rinçage à l'éthanol et concentration à sec, le résidu ainsi obtenu est traité avec de l'éther diéthylique. Après agitation dans l'éther diéthylique, le solide est filtré. On obtient après séchage une poudre de couleur blanche.
RMN ¹H (δ ppm, DMSO) : 3-4 (pic large, 2H) ; 5,10 (s, 2H) ; 6,57-7,42 (m, 10H); 8-9 (s large, 1H).

### 22.3) 4-fluoro-N-{4-[2-(phénoxyméthyl)-1H-imidazol-4-yl]phényl}benzamide

140 mg d'acide 4-fluoro-benzoïque (0,001mol) sont dissous dans du dichlorométhane (5 ml). Le chlorure d'oxalyle (0,13 ml, 0,0015 mol) est ajouté suivi d'une goutte de diméthylformamide. Après une agitation pendant trente minutes, on évapore à sec. Dans un ballon de 25 ml, 265 mg de 4-[2-(phénoxyméthyl)-1H-imidazol-4-yl]aniline (0,001 mol) sont dissous dans du dichlorométhane (5 ml). 0,15 ml de triéthylamine (0,0011 mol) puis le dérivé chlorure d'acide (obtenu précédemment) dilué dans 3 ml de dichlorométhane sont rajoutés. Après une agitation pendant 20 heures à 23° C, on évapore à sec. 30 ml d'eau et 30 ml d'acétate d'éthyle sont rajoutés. Après décantation, la phase aqueuse est extraite avec de l'acétate d'éthyle. La phase organique est lavée avec une solution saturée en chlorure de sodium, séchée sur sulfate de sodium et le solvant est évaporé. Le résidu obtenu est adsorbé sur silice puis purifié par chromatographie sur colonne de silice de type Biotage (éluant : acétate d'éthyle-heptane : 4-6). On obtient une poudre de couleur crème.
RMN ¹H (δ ppm, DMSO) : 5,08 (s, 2H); 6,94-8,06 (m, 14H); 10,23 (s, 1H); 12,37 (s large, 1H)
MH+ expérimental = 388,20 ; MH+ théorique = 387,41
Point de fusion : 224-225° C

### Exemple 23 : 4-{4-[2-(phénoxyméthyl)-1H-imidazol-4-yl]phénoxy}benzonitrile

Ce composé est synthétisé selon une méthode analogue à celle décrite à l'exemple 1.
MH+ expérimental = 368,20 ; MH+ théorique = 367,41
Point de fusion : 179-181°C

### Exemple 24 : 4-[2-(phénoxyméthyl)-1H-imidazol-4-yl] benzoate d'éthyle

### 24.1) 4-(bromoacétyl)benzoate d'éthyle

Ce composé est préparé selon une méthode analogue à celle décrite à l'exemple 1.2 en utilisant comme composé de départ le 4-acétylbenzoate d'éthyle (4 g, 0,020 mol). On obtient une poudre de couleur crème.

RMN ¹H (δ ppm, DMSO) : 1,33 (t, 3H) ; 4,34 (q, 2H) ; 4,98 (s, 2H) ; 8,07-8,12 (m, 4H)

### 24.2) 4-[2-(phénoxyméthyl)-1H-imidazol-4-yl]benzoate d'éthyle

Ce composé est préparé selon une méthode analogue à celle décrite à l'exemple 1.3 en utilisant comme composés de départ le 4-(bromoacétyl)benzoate d'éthyle (2 g, 0,0074 mol) et le chlorhydrate de 2-phénoxyéthanimidamide (1,6 g, 0,0086 mol). Après traitement, on obtient une poudre de couleur blanche.
RMN ¹H (δ ppm, DMSO) : 1,32 (t, 3H) ; 4,31 (q, 2H) ; 5,27 (s, 2H) ; 7,00-7,36 (m, 5H); 7,93-8,07 (m, 5H)
MH+ expérimental = 323,20 ; MH+ théorique = 322,36
Point de fusion: 133-135° C

### Exemple 25 : 4-{4-[2-(phénoxyméthyl)-1H-imidazol-4-yl]phénoxy}benzoate d'éthyle

Ce composé est synthétisé selon une méthode analogue à celle décrite à l'exemple 1.
MH+ expérimental = 415,20 ; MH+ théorique = 414,46
Point de fusion : 100-102° C

### Exemple 26 : 4-{4-[4-(méthylthio)phénoxy]phényl}-2-(phénoxyméthyl)-1H-imidazole

Ce composé est synthétisé selon une méthode analogue à celle décrite à l'exemple 1.
MH+ expérimental = 389,10 ; MH+ théorique = 388,49
Point de fusion : 122-124° C

### Exemple 27 : 4-{4-[4-(méthylsulfonyl)phénoxy]phényl}-2-(phénoxyméthyl)-1H-imidazole

### 27.1) 1-{4-[4-(méthylthio)phénoxy]phényl}éthanone

Ce composé est préparé selon une méthode analogue à celle décrite à l'exemple 1.1 en utilisant comme composés de départ le 4-thiométhoxyphénol (10,35 g, 0,0725 mol) et la 4-fluoroacétophénone (10 g, 0,0725 mol). Après traitement, on obtient une poudre de couleur beige.
RMN ¹H (δ ppm, DMSO) : 2,49 (s, 3H) ; 2,53 (s, 3H) ; 7,03 (d, 2H) ; 7,07 (d, 2H) ; 7,34 (d, 2H) ; 7,96 (d, 2H)

### 27.2) 2-bromo-1-{4-[4-(méthylthio)phénoxy]phényl}éthanone

Ce composé est préparé selon une méthode analogue à celle décrite à l'exemple 1.2 en utilisant comme composé de départ le 1-{4-[4-(méthylthio)phénoxy]phényl}éthanone (11,9 g, 0,046 mol) ; on obtient une poudre de couleur blanche.
RMN ¹H (δ ppm, DMSO) : 2,50 (s, 3H) ; 4,85 (s, 2H) ; 7,05 (d, 2H) ; 7,10 (d, 2H) ; 7,35 (d, 2H) ; 8,01 (d, 2H)

### 27.3) 4-{4-[4-(méthylthio)phénoxy]phényl}-2-(phénoxyméthyl)-1H-imidazole

Ce composé est préparé selon une méthode analogue à celle décrite à l'exemple 1.3 en utilisant comme composés de départ l'acide phénoxyacétique (3,92 g, 0,0253 mol) et la 2-bromo-1-{4-[4-(méthylthio)phénoxy]phényl} éthanone (8,53 g, 0,0253 mol). Après traitement, on obtient une poudre de couleur blanche.
RMN ¹H (δ ppm, DMSO): 2,46 (s, 3H) ; 5,07 (s, 2H) ; 6,94-7,07 (m, 7H) ; 7,28-7,77 (m, 8H)
MH+ expérimental = 389,10 ; MH+ théorique = 388,48.

### 27.4) 4-{4-[4-(méthylsulfonyl)phénoxy]phényl}-2-(phénoxyméthyl)-1H-imidazole

207 mg de 4-{4-[4-(méthylthio)phénoxy]phényl}-2-(phénoxyméthyl)-1H-imidazole (0,00053 mol) sont solubilisés dans de l'acide acétique. Une solution d'eau oxygénée (0,4 ml d'une solution diluée à 30 % dans l'eau) est ajoutée goutte à goutte. On agite vingt heures à 23° C puis on évapore à sec. Le résidu est repris avec de l'eau et de l'acétate d'éthyle. Après décantation, la phase aqueuse est extraite avec de l'acétate d'éthyle. La phase organique est séchée sur sulfate de sodium puis évaporée à sec. Le résidu obtenu est adsorbé sur silice puis purifié par chromatographie sur colonne de silice de type Biotage (éluant : acétate d'éthyle-heptane : 2-1). On obtient une poudre de couleur blanche.
RMN ¹H (δ ppm, DMSO): 3,18 (s, 3H) ; 5,09 (s, 2H) ; 6,90-7,33 (m, 10H) ; 7,65-7,91 (m, 4H) ; 12,43 (sé, 1H)
MH+ expérimental = 421,10 ; MH+ théorique = 420,49.
Point de fusion : 143-145° C

### Exemple 28 : chlorhydrate de 4-{4-[2-(phénoxyméthyl)-1H-imidazol-4-yl] phénoxy}aniline

### 28.1) 2-bromo-1-[4-(4-nitrophénoxy)phényl]éthanone

Ce composé est préparé selon une méthode analogue à celle décrite à l'exemple 1.2 en utilisant comme composé de départ le 4-acétyl-4' nitrodiphényl éther (15g, 0,0566 mol) ; on obtient une poudre de couleur blanche.
RMN ¹H (δ ppm, DMSO) : 4,93 (s, 2H) ; 7,27-7,31 (m, 4H) ; 8,10 (d, 2H) ; 8,30 (d, 2H)
28.2) 4-[4-(4-nitrophénoxy)phényl]-2-(phénoxyméthyl)-1H-imidazole

Ce composé est préparé selon une méthode analogue à celle décrite à l'exemple 1.3 en utilisant comme composés de départ l'acide phénoxyacétique (8,14 g, 0,053 mol) et la 2-bromo-1-[4-(4-nitrophénoxy)phényl]éthanone (18 g, 0,053 mol). Après traitement, on obtient une poudre de couleur beige.
MH+ expérimental = 388,20 ; MH+ théorique = 387,39.

### 28.3) chlorhydrate de 4-{4-[2-(phénoxyméthyl)-1H-imidazol-4-yl]phénoxy}aniline

Dans un réacteur de 11, 3,92 g de 4-[4-(4-nitrophénoxy)phényl]-2-(phénoxyméthyl)-IH-imidazole (0,0101 mol) sont hydrogénés pendant 7 heures sous une pression d'hydrogène de 1,5 bars avec une quantité catalytique de palladium adsorbé sur charbon (10 % massique) dans de l'éthanol (50 ml). On filtre sur filtre millipore puis on rince à l'éthanol. Après une concentration à sec, le résidu est trituré avec de l'éther diéthylique et le mélange est agité dans l'éther diéthylique puis le solide est filtré. Le produit obtenu est adsorbé sur silice puis purifié par chromatographie sur colonne de silice de type Biotage (éluant : acétate d'éthyle-heptane : 5-5 à 7-3). On obtient une poudre de couleur beige.
MH+ expérimental = 358,20 ; MH+ théorique = 357,41.

Le solide obtenu est mis en suspension dans 100 ml d'éthanol. De l'acide chlorhydrique dilué dans l'éther diéthylique (8,4 ml d'une solution 1 N dans l'éther diéthylique) est ajouté à ce mélange refroidi à 0° C. Après agitation pendant une heure, on évapore à sec puis on reprend à l'éther diéthylique et on filtre. Après séchage, on obtient une poudre de couleur beige.
RMN ¹H (δ ppm, DMSO) : 3,7-4 (sé) ; 5,45 (s, 2H) ; 7,01-7,40 (m, 11H) ; 7,93 (d, 2H), 8,14 (s, 1H) ; 11-13 (pic large, 2H)
Point de fusion : >300° C

### Exemple 29 : trifluoroacétate de {4-[2-(phénoxyméthyl)-1H-imidazol-4-yl]phényl} phényl méthanone

### 29.1) 1-(4-benzoylphényl)éthanone

Un mélange contenant l'acide 4-acétyl benzoïque (6 g , 0,0365 mol), l'acide boronique (5,34 g, 0,044 mol), l'acétate de palladium (245 mg, 0,001 mol), la tricyclohexyl phosphine (716 mg, 0,0025 mol) et l'anhydride pivalique (11,2 ml, 0,054 mol) dans un volume de solvants eau-tetrahydrofuranne : 1,6 ml-130 ml est chauffé à 60° C sous argon pendant 20 heures. Après concentration à sec, le résidu obtenu est adsorbé sur silice puis purifié par chromatographie sur colonne de silice de type Biotage (éluant : acétate d'éthyle-heptane : 9-1 à 8-2). On obtient une poudre de couleur blanche.
RMN ¹H (δ ppm, DMSO) : 2,65 (s, 3H) ; 7,56-8,11 (m, 9H)

### 29.2) 1-(4-benzoylphényl)-2-bromoéthanone

Ce composé est préparé selon une méthode analogue à celle décrite à l'exemple 1.2 en utilisant comme composé de départ le 1-(4-benzoylphényl)éthanone (880 mg, 0,0039 mol) ; on obtient une poudre de couleur blanche.

RMN ¹H (δ ppm, DMSO) : 5,08 (s, 2H) ; 7,56-8,16 (m, 9H)

### 29.3) trifluoroacétate de {4-[2-(phénoxyméthyl)-1H-imidazol-4-yl]phényl}phényl méthanone

Ce composé est préparé selon une méthode analogue à celle décrite à l'exemple 21.3 en utilisant comme composés de départ le composé 2-phénoxyéthanimidamide (400 mg, 0,00266 mol) et la 1-(4-benzoylphényl)-2-bromoéthanone (685 mg, 0,00226 mol). Après traitement et passage du résidu obtenu sur colonne de silice RP18 (éluant: acétonitrile-acide trifluoroacétique 0,1N : 5-5), on obtient une poudre de couleur beige.
RMN ¹H (δ ppm, DMSO) : 3-5 (pic très large) ; 5,25 (s, 2H) ; 6,98-8,05 (m, 15H)
MH+ expérimental = 355,20 ; MH+ théorique = 354,41
Point de fusion : < 50° C

### Exemple 30 : trifluoroacétate de N-(4-fluorophényl)-4-[2-(phénoxyméthyl)-1H-imidazol-4-yl]benzamide

### 30.1) 4-acétyl-N-(4-fluorophényl)benzamide

Un mélange contenant de l'acide 4-acétyl-benzoïque (4 g, 0,0243 mol), de l'hydrochlorure de 1-(3-diméthylaminopropyl)-3-éthylcarbodiimide (5,13 g, 0,02673 mol), de la triéthylamine (3,7 ml, 0,02673 mol), de la 1-hydroxybenzotriazole (3,62 g, 0,02673 mol) et de la 4-fluoroaniline (2,81 ml ; 0,02916 mol) dans 60 ml de tétrahydrofuranne est agité pendant 48 heures à 23°C. On filtre sur fritté et on évapore à sec. Le résidu est repris dans un mélange de solvants : acétate d'éthyle-eau : 50-50. Le précipité est filtré sur fritté, lavé à l'éther isopropylique puis à l'isopentane. Après séchage, on obtient une poudre de couleur blanche.
RMN ¹H (δ ppm, DMSO) : 2,72 (s, 3H) ; 7,17-7,22 (m, 2H) ; 7,77-7,81 (m, 2H) ; 8,05-8,09 (m, 4H) ; 10,46 (s, 1H)
30.2) 4-(bromoacétyl)-N-(4-fluorophényl)benzamide

La 4-acétyl-N-(4-fluorophényl)benzamide (1,5 g, 0,00583 mol) est dissoute dans 100 ml de méthanol. On ajoute la résine tribromure de pyridinium (4 g de résine à 2 mmol de Br₃ par gramme, 0,0081 mol) et on chauffe pendant 4 heures à 40° C. On filtre sur fritté, rince au méthanol et évapore à sec. On obtient une poudre de couleur jaune pâle.
RMN ¹H (δ ppm, DMSO) : 4,99 (s, 2H) ; 7,18-7,23 (m, 2H) ; 7,78-7,81 (m, 2H) ;
8,06-8,14 (m, 4H) ; 10,47 (s, 1H)

### 30.3) trifluoroacétate de N-(4-fluorophényl)-4-[2-(phénoxyméthyl)-1H-imidazol-4-yl]benzamide

Ce composé est préparé selon une méthode analogue à celle décrite à l'exemple 21.3 en utilisant comme composés de départ la 2-phénoxyéthanimidamide (225 mg, 0,0015 mol) et la 4-(bromoacétyl)-N-(4-fluorophényl)benzamide (426 mg, 0,00128 mol). Après traitement et passage du résidu obtenu sur colonne de silice RP18 (éluant : acétonitrile-acide trifluoroacétique 0,1N : 5-5), on obtient une poudre de couleur jaune.
RMN ¹H (δ ppm, DMSO) : 3-4 (pic large) ; 5,28 (s, 2H) ; 7,01-7,37 (m, 7H) ; 7,78-8,02 (m, 7H) ; 10,30 (s, 1H)
MH+ expérimental = 388,10 ; MH+ théorique = 387,41
Point de fusion : 198-200° C

### Exemple 31 : 4-[4-(3-nitrophénoxy)phényl]-2-(phénoxyméthyl)-1H-imidazole

### 31.1) 1-nitro-3-phénoxybenzène

Ce composé est préparé selon une méthode analogue à celle décrite à l'exemple 1.1 en utilisant comme composés de départ le 1-fluoro-3-nitro benzène (15,4 g, 0,106 mol) et le phénol (10 g, 0,106 mol). Après traitement, on obtient une huile de couleur orange.

RMN ¹H (δ ppm, DMSO) : 7,13-7,99 (m, 9H)
31.2) 1-[4-(3-nitrophénoxy)phényl]éthanone

Le composé 1-nitro-3-phénoxybenzène (10,17 g, 0,0473 mol) est mis en solution dans du disulfure de carbone (70 ml). Le chlorure d'aluminium (10,06 g, 0,076 mol) est ajouté par portion à température ambiante. On refroidit à 0° C puis on ajoute goutte à goutte le chlorure d'acétyle (2,4 ml, 0,038 mol). On laisse remonter à 23° C puis l'agitation est maintenue pendant 5 heures. On refroidit de nouveau à 0° C puis on ajoute prudemment de l'acétate d'éthyle, de la glace pilée et de l'acide chlorhydrique 3N. Après décantation, on extrait par l'acétate d'éthyle. La phase organique est lavée à l'eau, avec une solution saturée en carbonate de sodium puis avec une solution saturée en chlorure de sodium. Elle est ensuite séchée sur sulfate de sodium puis évaporée. Le résidu obtenu est adsorbé sur silice puis purifié par chromatographie sur colonne de silice de type Biotage (éluant: acétate d'éthyle-heptane :1-4). Après un lavage à l'éther diéthylique, on obtient une poudre de couleur jaune.
RMN 1H (δ ppm, DMSO) : 2,57 (s, 3H) ; 7,20 (d, 2H) ; 7,50-8,04 (m, 6H)

### 31.3) 2-bromo-1-[4-(3-nitrophénoxy)phényl]éthanone

Ce composé est préparé selon une méthode analogue à celle décrite à l'exemple 1.2 en utilisant comme composé de départ le 1-[4-(3-utrophénoxy)phényl]éthanone (5,4 g, 0,021 mol) ; on obtient une poudre de couleur beige.
RMN ¹H (δ ppm, DMSO) : 4,92 (s, 2H) ; 7,21-7,23 (m, 2H) ; 7,64-8,08 (m, 6H)

### 31.4) 4-[4-(3-nitrophénoxy)phényl]-2-(phénoxyméthyl)-1H-imidazole

Ce composé est préparé selon une méthode analogue à celle décrite à l'exemple 1.3 en utilisant comme composés de départ l'acide phénoxyacétique (1,16 g, 0,0075 mol) et le composé 2-bromo-1-[4-(3-nitrophénoxy)phényl]éthanone (2,46 g, 0,0075 mol). Après traitement, on obtient une poudre de couleur beige.
RMN ¹H (δ ppm, DMSO) : 5,08 (s, 2H) ; 6,94-7,87 (m, 14H) ; 12,45 (sé, 1H)
MH+ expérimental = 388,20 ; MH+ théorique = 387,39.
Point de fusion : 140-142° C

### Exemple 32 : chlorhydrate de 3-{4-[2-(phénoxyméthyl)-1H-imidazol-4-yl] phénoxy}aniline

Dans un réacteur de 250 ml, la 4-[4-(3-nitrophénoxy)phényl]-2-(phénoxyméthyl)-1H-imidazole (0,28 g, 0,00072 mol) est hydrogénée pendant 1 heure sous une pression d'hydrogène de 1,5 bars avec une quantité catalytique de palladium adsorbé sur charbon (10 % massique) dans de l'éthanol (70 ml).

On filtre sur filtre millipore puis on rince à l'éthanol. Après concentration à sec, le résidu est trituré avec de l'éther diéthylique, puis agité dans un mélange d'éther diéthylique et d'isopentane (1:9) et enfin le solide est filtré. Après séchage, on obtient une poudre de couleur jaune qui est purifiée par chromatographie sur colonne de silice RP18 (éluant : acétonitrile-acide trifluoroacétique 0,1N : 1-2).
RMN ¹H (δ ppm, DMSO) : 3-4 (sé) ; 5,30 (s, 2H) ; 6,21-6,26 (m, 2H) ; 6,39 (d, 1H) ; 7,01-7,09 (m, 6H) ; 7,33-7,37 (m, 2H) ; 7,76-7,78 (m, 2H) ; 7,92 (s, 1H)
MH+ expérimental = 358,20 ; MH+ théorique = 357,41

Le solide obtenu à l'étape précédente (0,07 g, 0,00020 mol) est agité pendant 30 minutes dans un mélange d'acétate d'éthyle et une solution saturée en hydrogénocarbonate de sodium. On laisse décanter puis la phase organique est lavée avec une solution saturée en carbonate de sodium, séchée sur sulfate de sodium puis évaporée. Le résidu est repris dans l'éthanol (7 ml) puis, à 0° C, une solution 1N d'acide chlorhydrique dans l'éther diéthylique (0,43 ml, 0,00044 mol) est ajoutée. Après agitation pendant 30 minutes, on évapore à sec puis on reprend à l'éther diéthylique et on filtre. Après séchage, on obtient une poudre de couleur beige.
RMN ¹H (δ ppm, DMSO) : 3-4 (sé) ; 5,44 (s, 2H) ; 6,67 (sé, 2H) ; 6,80 (sé, 1H) ; 7,02-7,38 (m, 8H) ; 7,89-7,91 (m, 2H) ; 8,14 (s, 1H)
MH+ expérimental = 358,20 ; MH+ théorique =357,41
Point de fusion : > 300° C

### Exemple 33 : 4-{4-[4-(benzyloxy)phénoxy]phényl}-2-(phénoxyméthyl)-1H-imidazole

Ce composé est synthétisé selon une méthode analogue à celle décrite à l'exemple 1.
MH+ expérimental = 449,20 ; MH+ théorique = 448,52
Point de fusion : 134-136° C

### Exemple 34 : 4-{4-[2-(phénoxyméthyl)-1H-imidazol-4-yl]phénoxy}phénol

### 34.1) 4-{4-[4-(benzyloxy)phénoxy]phényl}-2-(phénoxyméthyl)-1H-imidazole

Ce composé est préparé selon une méthode analogue à celle décrite à l'exemple 1.3 en utilisant comme produits de départ l'acide phénoxyacétique (1,82 g, 0,012 mol) et la 1-{4-[4-(benzyloxy)phénoxy]phényl}-2-bromoéthanone (4,76 g, 0,012 mol). Après traitement, on obtient une poudre de couleur beige.
RMN ¹H (δ ppm, DMSO) : 5,07 (s, 4H) ; 6,89-7,75 (m, 19H) ; 12,38 (s, 1H)
MH+ expérimental = 449,20 ; MH+ théorique = 448,5.
34.2) 4- {4-[2-(phénoxyméthyl)-1H-imidazol-4-yl]phénoxy}phénol

Dans un réacteur de 100 ml, 138 mg de 4-{4-[4-(benzyloxy)phénoxy]phényl}-2-(phénoxyméthyl)-1H-imidazole (0,0003 mol) dans 10 ml d'éthanol avec une quantité catalytique de palladium adsorbé sur charbon (10 % massique), sont hydrogénés pendant 24 heures sous une pression d'hydrogène de 4 bars. On filtre sur filtre millipore puis on rince à l'éthanol et on évapore à sec. Le résidu est trituré avec de l'éther diéthylique et l'ensemble est agité dans l'éther diéthylique et le solide est filtré. Le produit obtenu est adsorbé sur silice puis purifié par chromatographie sur colonne de silice de type Biotage (éluant : acétate d'éthyle-heptane : 2-8 à 5-5). Après un lavage à l'éther diéthylique, on obtient une poudre de couleur blanche.
RMN ¹H (δ ppm, DMSO) : 5,06 (s, 2H); 6,76-7,70 (m, 14H); 9,34 (s, 1H); 12,41 (sé, 1H)
MH+ expérimental = 359,20 ; MH+ théorique = 358,39
Point de fusion : 211-213° C

### Exemple 35 : 4-[4-(3-fluorophénoxy)phényl]-2-(phénoxyméthyl)-1H-imidazole

Ce composé est synthétisé selon une méthode analogue à celle décrite à l'exemple 1.
MH+ expérimental = 361,20 ; MH+ théorique = 360,39
Point de fusion : 177-179° C

### Exemple 36 : N-(4-{4-[2-(phénoxyméthyl)-1H-imidazol-4-yl]phénoxy}phényl) acétamide

La 4-{4-[2-(phénoxyméthyl)-1H-imidazol-4-yl]phénoxy}aniline (600 mg, 0,00168 mol), (préparée selon une méthode analogue à celle décrite à l'exemple 28) est mise en suspension dans l'éthanol (30 ml) à 0° C. La triéthylamine (0,235 ml, 0,00168 mol) et l'iodure de méthyle (0,136 ml, 0,0022 mol) sont ajoutés. Le mélange est agité 20 heures puis évaporé à sec. Le résidu obtenu est adsorbé sur silice puis purifié par chromatographie sur colonne de silice de type Biotage (éluant : acétate d'éthyle-heptane : 4-6 à 5-5). Après un lavage à l'éther diéthylique et à l'isopentane, on obtient une poudre de couleur beige.

RMN ¹H (δ ppm, DMSO) : 2,66 (s, 3H) ; 5,06 (s, 2H) ; 5,54 (sé, 1H) ; 6,54-7,70 (m, 14H) ; 12,32 (sé, 1H)
MH+ expérimental = 372,30 ; MH+ théorique = 371,44
Point de fusion : 176-177° C

### Exemple 37 : trifluoroacétate de 2-nitro-4-{4-[2-(phénoxyméthyl)-1H-imidazol-4-yl]phénoxy}aniline

Ce composé est synthétisé selon une méthode analogue à celle décrite à l'exemple 1.
MH+ expérimental = 403,20 ; MH+ théorique = 402,41
Point de fusion : 178-180° C

### Exemple 38 : N-méthyl-N-(4-{4-[2-(phënoxyméthyl)-1H-imidazol-4-yl]phénoxy}phényl) amine

La 4-{4-[2-(phénoxyméthyl)-1H-imidazol-4-yl]phénoxy}aniline (238 mg, 0,0066 mol), (préparée selon une méthode analogue à celle décrite à l'exemple 28) est mise en suspension dans du dichlorométhane (5 ml) à 23° C. La triéthylamine (0,1 ml, 0,0079 mol) et l'anhydride acétique (0,062 ml, 0,0066 mol) sont ajoutés. Le mélange est alors agité 20 heures puis évaporé à sec. L'huile obtenue est reprise par de l'acétate d'éthyle et de l'eau. Après décantation et extraction par l'acétate d'éthyle, la phase organique est lavée à l'eau puis avec une solution saturée en chlorure de sodium, séchée sur sulfate de sodium puis le solvant est évaporé. Le résidu obtenu est adsorbé sur silice puis purifié par chromatographie sur colonne de silice de type Biotage (éluant : acétate d'éthyle-heptane : 7-3 à 9-1). Après un lavage à l'éther diisopropylique et à l'isopentane, on obtient une poudre de couleur beige.
RMN ¹H (δ ppm, DMSO) : 2,03 (s, 3H) ; 5,08 (s, 2H) ; 6,94-7,74 (m, 14H) ; 9,92 (s, 1H) ; 12,32 (sé, 1H) MH+ expérimental = 400,30 ; MH+ théorique = 399,44
Point de fusion : 125-126° C

### Exemple 39 : 3-{4-[2-(phénoxyméthyl)-1H-imidazol-4-yl]phénoxy}benzonitrile

Ce composé est synthétisé selon une méthode analogue à celle décrite à l'exemple 1.
MH+ expérimental = 368,30 ; MH+ théorique = 367,41
Point de fusion : 161-163° C

### Exemple 40 : 4-[4-(2-nitrophénoxy)phényl]-2-(phénoxyméthyl)-1H-imidazole

Ce composé est synthétisé selon une méthode analogue à celle décrite à l'exemple 31.
MH+ expérimental = 388,20 ; MH+ théorique = 387,39
Point de fusion : 128-129° C

### Exemple 41 : chlorhydrate de 2-{4-[2-(phénoxyméthyl)-1H-imidazol-4-yl] phénoxy}aniline

Ce composé est synthétisé selon une méthode analogue à celle décrite à l'exemple 31.
MH+ expérimental = 358,30 ; MH+ théorique = 357,41
Point de fusion : 88-89° C

### Exemple 42 : chlorhydrate de 1-(4-{4-[2-(phénoxyméthyl)-1H-imidazol-4-yl] phénoxy}phényl)méthanamine

Dans un réacteur de 100 ml, 120 mg de 4-{4-[2-(phénoxyméthyl)-1H-imidazol-4-yl]phénoxy}benzonitrile (0,00033 mol) (obtenu selon une méthode analogue à celle décrite à l'exemple 1) dans de l'éthanol (10 ml) en présence d'acide chlorhydrique évaporé (0,1 ml) et d'une quantité catalytique de palladium adsorbé sur charbon (10 % massique), sont hydrogénés pendant deux jours sous une pression d'hydrogène de 1,5 bars. On filtre sur filtre millipore puis on rince à l'éthanol. On évapore à sec et le résidu est trituré avec un mélange de solvants tels que l'éther diéthylique et l'éthanol en proportion 1-1. On obtient après séchage une poudre de couleur verte.
RMN ¹H (δ ppm, DMSO) : 4,00 (m, 2H); 5,36 (s, 2H); 7,00-8,32 (m, 17H)
MH+ expérimental = 372,30 ; MH+ théorique = 371,44
Point de fusion : > 300° C

### Exemple 43 : chlorhydrate de 1-(3-{4-[2-(phénoxyméthyl)-1H-imidazol-4-yl] phénoxy}phényl)méthanamine

Ce composé est synthétisé selon une méthode analogue à celle décrite à l'exemple 42.
MH+ expérimental = 272,30 ; MH+ théorique = 271,44
Point de fusion : 200-202° C

### Exemple 44 : 4-[4-(3-bromophénoxy)phényl]-2-(phénoxyméthyl)-1H-imidazole

Ce composé est synthétisé selon une méthode analogue à celle décrite à l'exemple 1.

MH+ expérimental = 421,10 ; MH+ théorique = 421,29
Point de fusion : 133-135° C

### Exemple 45 : chlorhydrate de 2-fluoro-4-{4-[2-(phénoxyméthyl)-1H-imidazol-4-yl] phénoxy}aniline

### 45.1) 4-amino-3-fluorophénol

Dans un réacteur de 1 l, 10 g de 4-nitro-3-fluorophénol (0,0637 mol) avec une quantité catalytique de palladium adsorbé sur charbon (10 % massique) dans de l'éthanol (250 ml)est hydrogéné pendant 2 heures sous une pression d'hydrogène de 1,5 bars. Puis on filtre sur filtre millipore puis on rince à l'éthanol. On évapore à sec et le résidu est trituré avec de l'éther diéthylique et on agite le tout dans un mélange d'éther diisopropylique et d'isopentane (1:4). Après filtration du solide et séchage, on obtient une poudre de couleur beige.
RMN¹H (δ ppm, DMSO) : 4,35 (s, 2H) ; 6,32-6,61 (m, 3H) ; 8,75 (s, 1H)
45.2) 1-[4-(4-amino-3-fluorophénoxy)phényl]éthanone

Ce composé est préparé selon une méthode analogue à celle décrite à l'exemple 1.1 en utilisant comme composés de départ le 4-amino-3-fluorophénol (7,68 g, 0,06 mol) et la 4-fluoroacétophénone (8,34 g, 0,06 mol). Après traitement, on obtient une poudre de couleur blanche.
RMN ¹H (δ ppm, DMSO) : 2,51 (s, 3H) ; 5,10 (s, 2H) ; 6,69-6,96 (m, 5H) ; 7,92 (d, 2H)

### 45.3) chlorhydrate de 1-[4-(4-amino-3-fluorophénoxy)phényl]-2-bromoéthanone

Ce composé est préparé selon une méthode analogue à celle décrite à l'exemple 1.2 en utilisant comme composés de départ la 1-[4-(4-amino-3-fluorophénoxy) phényl]éthanone (4 g, 0,0163 mol) ; on obtient une poudre de couleur rose.
RMN ¹H (δ ppm, DMSO) : 4,84 (s, 2H) ; 5-6 (pic large) ; 6,99-7,13 (m, 5H) ; 7,93-8,02 (m, 2H)

### 45.4) chlorhydrate de 2-fluoro-4-{4-[2-(phénoxyméthyl)-1H-imidazol-4-yl] phénoxy} aniline

Ce composé est préparé selon une méthode analogue à celle décrite à l'exemple 1.3 en utilisant comme composés de départ l'acide phénoxyacétique (1,6 g, 0,0106 mol), et le chlorhydrate de 1-[4-(4-amino-3-fluorophénoxy)phényl]-2-bromoéthanone (4,2 g, 0,0106 mol) et du carbonate de césium (3,44 g, 0,0106 mol). Après traitement, on obtient une poudre de couleur beige.
MH+ expérimental = 376,20 ; MH+ théorique = 375,4

Le solide obtenu (0,06 g, 0,00016 mol) est mis en suspension dans 7 ml d'éthanol. A ce mélange refroidi à 0° C, on verse 0,35 ml d'une solution 1N d'acide chlorhydrique dans de l'éther diéthylique (0,00032 mol). Après agitation pendant une heure à cette température, on évapore à sec puis on reprend à l'éther diéthylique et on filtre. Après séchage, on obtient une poudre de couleur rose.
RMN ¹H (δ ppm, DMSO) : 3-5 (pic large); 5,44 (s, 2H) ; 6,77-6,80 (m, 1H); 6,97-7,12 (m, 7H) ; 7,34-7,38 (m, 2H) ; 7,86 (d, 2H) ; 8,11 (s, 1H)
Point de fusion : > 300° C

### Exemple 46 : 4-[4-(3-chlorophénoxy)phényl]-2-(phénoxyméthyl)-1H-imidazole

Ce composé est synthétisé selon une méthode analogue à celle décrite à l'exemple 1.
MH+ expérimental = 377,20 ; MH+ théorique = 376,84
Point de fusion : 146-148° C

### Exemple 47 : 4-[4-(3,5-difluorophénoxy)phényl]-2-(phénoxyméthyl)-1H-imidazole

Ce composé est synthétisé selon une méthode analogue à celle décrite à l'exemple 1.
MH+ expérimental = 379,10 ; MH+ théorique = 378,38
Point de fusion : 160-161° C

### Exemple 48 : 4-(4-benzylphényl)-2-(phénoxyméthyl)-1H-imidazole

Ce composé est synthétisé selon une méthode analogue à celle décrite à l'exemple 31.
MH+ expérimental = 341,20 ; MH+ théorique = 340,42
Point de fusion : 147-148° C

### Exemple 49 : 4-[4-(3-méthylphénoxy)phényl]-2-(phénoxyméthyl)-1H-imidazole

Ce composé est synthétisé selon une méthode analogue à celle décrite à l'exemple 1.
MH+ expérimental = 357,20 ; MH+ théorique = 356,42
Point de fusion : 160-162° C

### Exemple 50 : chlorhydrate de 4-[4-(2-chlorophénoxy)phényl]-2-(phénoxyméthyl)-1H-imidazole

Ce composé est synthétisé selon une méthode analogue à celle décrite à l'exemple 1.
MH+ expérimental = 377,10 ; MH+ théorique = 376,84
Point de fusion : 158-160° C

### Exemple 51 : 4-[4-(2-fluorophénoxy)phényl]-2-(phénoxyméthyl)-1H-imidazole

Ce composé est synthétisé selon une méthode analogue à celle décrite à l'exemple 1.
MH+ expérimental = 361,10 ; MH+ théorique = 360,39
Point de fusion : 154-156° C

### Exemple 52 : 4-[4-(3,4-difluorophénoxy)phényl]-2-(phénoxyméthyl)-1H-imidazole

Ce composé est synthétisé selon une méthode analogue à celle décrite à l'exemple 1.
MH+ expérimental = 379,10 ; MH+ théorique = 378,38
Point de fusion: 188-190° C

### Exemple 53 : chlorhydrate de N¹-(4-{4-[2-(phénoxyméthyl)-1H-imidazol-4-yl] phénoxy}phényl)glycinamide

### 53.1) tert-butyl {2-oxo-2-[(4-{4-[2-(phénoxyméthyl)-1H-imidazol-4-yl]phénoxy} phényl)amino]éthyl}carbamate

On agite pendant 24 heures à 23° C, un mélange contenant de l'acide terbutoxycarbonyl N-glycine (417 mg, 0,00238 mol), de l'hydrochlorure de 1-(3-diméthylaminopropyl)-3-éthylcarbodiimide (1 g, 0,00524 mol), de la triéthylamine (0,73 ml, 0,00524 mol), du 1-hydroxybenzotriazole (354 mg, 0,00262 mol) et de la {4-[2-(phénoxyméthyl)-1H-imidazol-4-yl]phénoxy}aniline (850 mg, 0,00238 mol, préparé selon l'exemple 28) dans 15 ml de diméthylformamide et 3 ml de dichlorométhane. On évapore à sec puis l'huile obtenue est reprise par de l'acétate d'éthyle et de l'eau. Après décantation et extraction par l'acétate d'éthyle, la phase organique est lavée à l'eau puis avec une solution saturée en chlorure de sodium, séchée sur sulfate de sodium puis le solvant est évaporé. Le résidu obtenu est adsorbé sur silice puis purifié par chromatographie sur colonne de silice de type Merck (éluant : dichlorométhane-méthanol : 98-2 à 95-5). Après un lavage à l'éther diisopropylique et à l'isopentane, on obtient une poudre de couleur orangée.
RMN ¹H (δ ppm, DMSO): 1,38 (s, 9H) ; 3,69-3,71 (m; 2H) ; 5,07 (s, 2H) ; 6,94-7,45 (m, 15H) ; 9,92 (s, 1H) ; 12,3-12,4 (sé, 1H)
MH+ expérimental = 515,30 ; MH+ théorique = 514,56

### 53.2) chlorhydrate de N1-(4-{4-[2-(phénoxyméthyl)-1H-imidazol-4-yl]phenoxy}phényl)glycinamide

Le composé *tert*-butyl {2-oxo-2-[(4-{4-[2-(phénoxyméthyl)-1H-imidazol-4-yl] phénoxy}phényl)amino]éthyl}carbamate (331 mg, 0,00064 mol) dans 6 ml d'acétate d'éthyle est agité pendant 2 heures à 23° C puis on évapore à sec. Après un lavage à l'éther et à l'isopentane, on obtient après filtration et séchage, une poudre de couleur beige.
RMN ¹H (δ ppm, DMSO) : 3,3-3,7 (sé, 3H) ; 3,74-3,80 (m, 2R) ; 5,43 (s, 2H) ; 7,01-8,26 (m, 16H) ; 10, 84 (s, 1H) MH+ expérimental = 415,20 ; MH+ théorique = 414,46
Point de fusion : > 250° C

### Exemple 54 : 4-[4-(2,5-difluorophénoxy)phényl]-2-(phénoxyméthyl)-1H-imidazole

Ce composé est synthétisé selon une méthode analogue à celle décrite à l'exemple 1.
MH+ expérimental = 379,20 ; MH+ théorique = 378,38
Point de fusion : 128-130° C

### Exemple 55 : 4-[4-(2,4-difluorophénoxy)phényl]-2-(phénoxyméthyl)-1H-imidazole

Ce composé est synthétisé selon une méthode analogue à celle décrite à l'exemple 1.
MH+ expérimental = 379,20 ; MH+ théorique = 378,38
Point de fusion : 131-133° C

### Exemple 56 : 4-[4-(2,3-difluorophénoxy)phényl]-2-(phénoxyméthyl)-1H-imidazole

Ce composé est synthétisé selon une méthode analogue à celle décrite à l'exemple 1.
MH+ expérimental = 379,00 ; MH+ théorique = 378,38
Point de fusion : 127-129° C

### Exemple 57 : 4-[4-(2,6-difluorophénoxy)phényl]-2-(phénoxyméthyl)-1H-imidazole

Ce composé est synthétisé selon une méthode analogue à celle décrite à l'exemple 1.
MH+ expérimental = 379,00 ; MH+ théorique = 378,38
Point de fusion : 143-145° C

Selon une méthode analogue à celle décrite dans les exemples 1 à 57, on peut également préparés les composés suivants :
*N*-{4-[2-(phénoxyméthyl)-1H-imidazol-4-yl]phényl}pyridin-4-amine;
4-{4-[2-(phénoxyméthyl)-1H-imidazol-4-yl]phénoxy}pyridine;
4-({4-[2-(phénoxyméthyl)-1H-imidazol-4-yl]phényl}thio)pyridine;
*N*-{4-[2-(phénoxyméthyl)-1H-imidazol-4-yl]phényl}-*N*-phénylamine;
*N*-{4-[2-(phénoxyméthyl)-1H-imidazol-4-yl]phényl}pyrimidin-2-amine;
2-(phénoxyméthyl)-4-[4-(3,4,5-triméthoxyphénoxy)phényl]-1H-imidazole;
*N*-{4-[2-(phénoxyméthyl)-1H-imidazol-4-yl]phényl}-1H-indol-4-amine;
*N*-{4-[2-(phénoxyméthyl)-1H-imidazol-4-yl]phényl}-1H-indol-6-amine;
*N*-{4-[2-(phénoxyméthyl)-1H-imidazol-4-yl]phényl}pyrazin-2-amine ;
*N*-{4-[2-(phénoxyméthyl)-1H-imidazol-4-yl]phényl}-4H-1,2,4-triazol-4-amine ;
*N*-{4-[2-(phénoxyméthyl)-1H-imidazol-4-yl]phényl}furan-2-amine ;
*N*-{4-[2-(phénoxyméthyl)-1H-imidazol-4-yl]phényl}furan-3-amine ;
*N*-{4-[2-(phénoxyméthyl)-1H-imidazol-4-yl]phényl}-*N*-thién-2-ylamine ; ou un sel pharmaceutiquement acceptable de ces derniers.

### Etude pharmacologique

L'affinité des composés de la présente invention est déterminée en appliquant la procédure expérimentale suivante :

Les différentes lignées cellulaires sont incubées à 37° C dans une atmosphère contenant 5 % de CO₂ (incubateurs Format Scientifique) dans du DMEM (milieu Eagle modifié par Dulbecco) à 4,5 g/l de glucose auquel on a rajouté 10 % de sérum de veau inactivé par la chaleur, 50 U/ml de pénicilline, 50 µg/ml de streptomycine et 2 mM de glutamine (Gibco).

L'inhibition de la prolifération cellulaire est mesurée par le test colorimétrique au WST (sel de tetrazolium, Boehringer Manheim, Meylan, France). Les cellules sont ensemencées dans des microplaques 96 puits (TPP) à raison de 2000 cellules par puits pour les HT29, 1300 pour les DU145 et 1200 pour les MIA-Pa-Ca-2 dans 95 µl de milieu de culture. 24 heures après l'ensemencement, 5 µl de drogues sont rajoutés à différentes concentrations (le produit est dissous dans du DMA à 10-2M puis il est dilué dans du milieu de culture). Les concentrations finales vont de 25 µM à 0,5 µM. Après 72 heures d'incubation, on rajoute 10 µl de WST par puits et la détermination de l'absorbance s'effectue à 450 nM 2 heures après (Victor, Perkin Elmer).

Chaque expérience est effectuée deux fois et est le résultat de la mesure de l'absorbance de huit puits. Pour chaque produit, la mesure de l'IC₅₀ correspondant à la concentration du produit qui entraîne 50 % d'inhibition de la croissance cellulaire, est déterminée par un calcul de régression linéaire (déviation linéaire, déviation de la linéarité et différence entre les expériences, programme de calcul TSAR) sur la partie linéaire de la sigmoïde.

Les valeurs de IC₅₀ obtenues pour la majorité des composés varient de 1 µM à 10 nM.

## Revendications

1. Composé de formule générale (I) 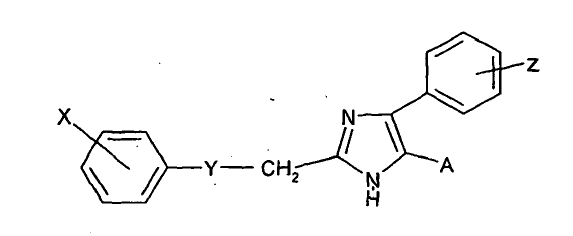 sous forme racémique, d'énantiomère ou toutes combinaisons de ces formes et dans laquelle :
X représente un ou plusieurs substituants identiques ou différents choisis parmi H et halo ;
Y représente -O- ou -S- ;
A représente H ou (C₁-C₆)alkyle ;
Z représente un ou plusieurs substituants identiques ou différents choisis parmi :
. (C₁-C₆)alkyle éventuellement substitué par un ou plusieurs radicaux halo identiques ou différents ;
. aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, -(CH₂)ₙ-NR₃R₄, (C₁-C₆)alkyl-sulfonyle, (C₁-C₆)alkyl-thio, (C₁-C₆)alkoxy éventuellement substitué par un ou plusieurs radicaux halo identiques ou différents, (C₁-C₆)alkoxy-carbonyle, phosphate, sulfate, glycoside et -NH-C(O)-CH(R_{A})-NR₅R₆ ;
. aryl-(C₁-C₆)alkyle ;
. hétéroaryle;
. -Z₁-Z'₁ ;
. -NR_{N}-C(O)-Z'₂ ; ou
. -Z₂-Z'₂;
Z₁ représente -O- ,-C(O)-O-, -NR_{N}-C(O)- ou -C(O)-NR_{N}- ;
Z'₁ représente un radical (C₁-C₁₀)alkyle ; aryle-(C₁-C₆)alkyle dont le radical aryle est éventuellement substitué par un ou plusieurs radicaux halo identiques ou différents ; ou (C₁-C₆)alkyle substitué par un ou plusieurs substituants choisis parmi : halo, (C₁-C₆)alkoxy, (C₁-C₆)alkylthio et -NR₁R₂ ;
R₁ et R₂ représentent, indépendamment, H ou (C₁-C₆)alkyle, ou bien forment ensemble avec l'atome d'azote sur lequel ils sont rattachés, un hétérocycloalkyle éventuellement substitué par (C₁-C₆)alkyle ;
Z₂ représente -O-, -S-, -SO₂-, -C(O)-, -C(O)-NR_{N}- ou -NR_{N}- ;
Z'₂ représente un radical aryle ou hétéroaryle, les radicaux aryle et hétéroaryle étant éventuellement substitués par un ou plusieurs radicaux identiques ou différents choisis parmi :
halo, nitro, cyano, hydroxy, (C₁-C₆)alkyle éventuellement substitué par un ou plusieurs radicaux halo identiques ou différents, (C₁-C₆)alkyl-thio, (C₁-C₆)alkyl-sulfonyle, (C₁-C₆)alkoxy éventuellement substitué par un ou plusieurs radicaux halo identiques ou différents, aryl-alkoxy, (C₁-C₆)alkoxy-carbonyle, phosphate, sulfate, glycoside, -(CH₂)ₙ-NR₃R₄ et NH-C(O)-CH(R_{A})-NR₅R₆ ;
R₃ et R₄ représentent, indépendamment, H, (C₁-C₆)alkyle, (C₁-C₆)alkyl-carbonyle ou (C₁-C₆)alkyl-sulfonyle, ou bien R₃ et R₄ forment ensemble avec l'atome d'azote sur lequel ils sont rattachés, un hétéroaryle ou un hétérocycloalkyle éventuellement substitué par (C₁-C₆)alkyle ;
R₅ et R₆ représentent, indépendamment, H ou (C₁-C₆)alkyle ;
R_{A} représente le résidu associé à l'acide aminé de formule NH₂-CH(R_{A})-C(O)-OH ;
R_{N} représente l'hydrogène ou un radical (C₁-C₆)alkyle ;
n représente un entier de 0 à 3 ; ou un sel pharmaceutiquement acceptable de ce dernier
à l'exclusion des composés dans lesquels A représente l'atome d'hydrogène et Z le radical -3-CF₃.

2. Composé selon la revendication 1, **caractérisé en ce que**
X représente un ou plusieurs substituants identiques ou différents choisis parmi H et halo ;
Y représente -O- ou -S- ;
A représente H ou (C₁-C₆)alkyle ;
Z représente un ou plusieurs substituants identiques ou différents choisis parmi :
. (C₁-C₆)akyle éventuellement substitué par un ou plusieurs radicaux halo identiques ou différents ;
. aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
. hétéroaryle ;
. - Z₁-Z'₁ ;
. -NH-C(O)-Z'₂ ; ou
. -Z₂-Z'₂ ;
Z₁ représente -O-, -NH-C(O)- ou -C(O)-NH- ;
Z'₁ représente un radical (C₄-C₁₀)alkyle ; aryle-(C₁-C₆)alkyle dont le radical aryle est éventuellement substitué par un ou plusieurs halo identiques ou différents ; ou (C₁-C₆)alkyle substitué par un ou plusieurs substituants choisis parmi halo, (C₁-C₆)alkoxy, (C₁-C₆)alkylthio et -NR₁R₂ ;
R₁ et R₂ représentent, indépendamment, H ou (C₁-C₆)alkyle, ou bien forment ensemble avec l'atome d'azote sur lequel ils sont rattachés, un hétérocycloalkyle éventuellement substitué par (C₁-C₆)alkyle ;
Z₂ représente -O-, -S-, -SO₂-, -C(O)- ou -C(O)-NH- ;
Z'₂ représente un radical aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents; ou un sel pharmaceutiquement acceptable de ce dernier.

3. Composé selon l'une des revendications 1 à 2, **caractérisé en ce que** A représente H et Y représente -O-; ou un sel pharmaceutiquement acceptable de ce dernier.

4. Composé selon l'une des revendications 1 à 3, **caractérisé en ce que** X représente H ; ou un sel pharmaceutiquement acceptable de ce dernier

5. Composé selon la revendication 4, **caractérisé en ce que**
Z représente un ou plusieurs substituants, identiques ou différents, en position méta et/ou para et choisis parmi hétéroaryle et -Z₂-Z'₂ ;
Z₂ représente -O-, -S-, -SO₂-, -C(O)- ou -C(O)-NH- ;
Z'₂ représente l'un des radicaux phényle ou naphtyle éventuellement substitués par un ou plusieurs radicaux identiques ou différents choisis parmi halo, nitro, cyano, hydroxy, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ; ou un sel pharmaceutiquement acceptable de ce dernier.

6. Composé selon l'une des revendications 1, 3 ou 4, **caractérisé en ce que** Z représente un hétéroaryle;
. -Z₁-Z'₁ dans lequel
soit Z₁ représente -O-, -NR_{N}-C(O)- ou -C(O)-NR_{N}- et Z'₁ représente le radical benzyle ;
soit Z₁ représente -O-, -C(O)-O-, -NR_{N}-C(O)- ou -C(O)-NR_{N}- et Z'₁ représente un radical (C₁-C₆)alkyle substitué par un ou plusieurs substituants choisis parmi : halo, (C₁-C₆)alkoxy, (C₁-C₆)alkylthio et -NR₁R₂ ;
R₁ et R₂ représentent, indépendamment, H ou (C₁-C₆)alkyle, ou bien forment ensemble avec l'atome d'azote sur lequel ils sont rattachés, un hétérocycloalkyle ;
. -Z₂-Z'₂ dans lequel
Z₂ représente -O-, -S-, -SO₂-, -C(O)-, -C(O)-NR_{N}- ou NR_{N}- ;
Z'₂ représente un radical phényle ou phényle substitué par un ou plusieurs radicaux identiques ou différents choisis parmi :
halo, nitro, cyano, hydroxy, (C₁-C₆)alkyle éventuellement substitué par un ou plusieurs radicaux halo identiques ou différents, (C₁-C₆)alkyl-thio, (C₁-C₆)alkyl-sulfonyle, (C₁-C₆)alkoxy éventuellement substitué par un ou plusieurs radicaux halo identiques ou différents, aryl-alkoxy, (C₁-C₆)alkoxy-carbonyle, -(CH₂)ₙ-NR₃R₄ et -NH-C(O)-CH(R_{A})-NR₅R₆;
R₃ et R₄ représentent, indépendamment, H, (C₁-C₆)alkyle ou (C₁-C₆)alkyl-carbonyle ;
R₅ et R₆ représentent, indépendamment, H ou (C₁-C₆)alkyle ;
R_{A} représente le résidu associé à l'acide aminé de formule NH₂-CH(R_{A})-C(O)-OH ;
R_{N} représente l'hydrogène ou un radical (C₁-C₆)alkyle ; ou un sel pharmaceutiquement acceptable de ce dernier.

7. Composé selon l'une des revendications 1, 3 ou 4, **caractérisé en ce que** Z représente un ou plusieurs substituants, identiques ou différents, de formule -Z₂-Z'₂ ; ou un sel pharmaceutiquement acceptable de ce dernier.

8. Composé selon la revendication 7, **caractérisé en ce que** Z est en position méta et/ou para ; ou un sel pharmaceutiquement acceptable de ce dernier.

9. Composé selon l'une des revendications 7 à 8, **caractérisé en ce que** Z₂ représente -O-, -S-, -SO₂- ou -C(O)- ; ou un sel pharmaceutiquement acceptable de ce dernier.

10. Composé selon la revendication 9, **caractérisé en ce que** Z₂ représente -O- ; ou un sel pharmaceutiquement acceptable de ce dernier.

11. Composé selon l'une des revendications 7 à 8, **caractérisé en ce que** Z₂ représente -NR_{N}-; ou un sel pharmaceutiquement acceptable de ce dernier.

12. Composé selon l'une des revendications 7 à 11, **caractérisé en ce que**
Z'₂ représente phényle ou phényle substitué par un ou plusieurs radicaux identiques ou différents choisis parmi :
halo, nitro, cyano, hydroxy, (C₁-C₆)alkyle éventuellement substitué par un ou plusieurs radicaux halo identiques ou différents, (C₁-C₆)alkyl-thio, (C₁C₆)alkyl-sulfonyle, (C₁-C₆)alkoxy éventuellement substitué par un ou plusieurs radicaux halo identiques ou différents, benzyloxy, (C₁-C₆)alkoxy-carbonyle, phosphate, -(CH₂)ₙ-NR₃R₄ et -NH-C(O)-CH(R_{A})-NR₅R₆ ;
R₃ et R₄ représentent, indépendamment, H, (C₁-C₆)alkyle, (C₁-C₆)alkyl-carbonyle ou (C₁-C₆)alkyl-sulfonyle ;
R_{N} représente l'hydrogène ou un radical (C₁-C₆)alkyle;
R₅ et R₆ représentent, indépendamment, H ou (C₁-C₆)alkyle ;
R_{A} représente le résidu associé à l'acide aminé de formule NH₂-CH(R_{A})-C(O)-OH ; ou un sel pharmaceutiquement acceptable de ce dernier.

13. Composé selon l'une des revendications 7 à 12 **caractérisé en ce que**
Z'₂ représente phényle substitué par un ou plusieurs radicaux identiques ou différents choisis parmi : halo, nitro, cyano, hydroxy, (C₁-C₆)alkyl-sulfonyle, (C₁₋C₆)alkoxy, -(CH₂)ₙ-NR₃R₄ et NH-C(O)-CH(R_{A})-NR₅R₆ ;
R₃ et R₄ représentent, indépendamment, H, (C₁-C₆)alkyle ou (C₁-C₆)alkyl-carbonyle ;
R₅ et R₆ représentent, indépendamment, H ou (C₁-C₆)alkyle; ou un sel pharmaceutiquement acceptable de ce dernier;

14. Composé selon la revendication 13 **caractérisé en ce que**
Z'₂ représente phényle substitué par au moins deux radicaux identiques ou différents choisis parmi : fluoro, nitro, cyano, hydroxy, (C₁-C₆)alkyl-sulfonyle, (C₁-C₆)alkoxy, -NH₂ et -NH-C(O)-CH(R_{A})-NR₅R₆; R₅ et R₆ représentent, indépendamment, H ou (C₁-C₆)alkyle ; ou un sel pharmaceutiquement acceptable de ce dernier.

15. Composé selon l'une des revendications 7 à 11, **caractérisé en ce que**
Z'₂ représente le radical pyridinyle, pyrimidinyle, pyrazinyle, triazolyle, furyle, thiényle, purinyle, triazinyle, pyrrazolo-pyrimidinyle, quinoxalinyle ou indolyle,
chacun de ces radicaux étant éventuellement substitué par un ou plusieurs radicaux identiques ou différents choisis parmi : halo, nitro, cyano, hydroxy, (C₁-C₆)alkyle et -NH₂ ; ou un sel pharmaceutiquement acceptable de ce dernier.

16. Composé selon la revendication 1 **caractérisé en ce qu'**il répond à l'une des formules suivantes :
4-[4-(4-fluorophénoxy)phényl]-2-(phénoxyméthyl)-1H-imidazole ;
4-(1,1'-biphényl-4-yl)-2-[(phénylthio)méthyl]-1H-imidazole ;
4-(1,1'-biphényl-4-yl)-2-(phénoxyméthyl)-1H-imidazole ;
4-[4-(4-fluorophénoxy)phényl]-2-[(phénylthio)méthyl]-1H-imidazole;
2-[(4-fluorophénoxy)méthyl]-4-[4-(4-fluorophénoxy)phényl]-1H-imidazole ;
2-(phénoxyméthyl)-4-[4-(phénylthio)phényl]-1H-imidazole ;
2-(phénoxyméthyl)-4-[4-(phénylsulfonyl)phényl]-1H-imidazole;
4-(4-[(2-fluorobenzyl)oxy]phényl)-2-(phénoxyméthyl)-1H-imidazole;
trifluoroacétate de 2-(phénoxyméthyl)-4-(4-phénoxyphényl)-1H-imidazole;
trifluoroacétate de 4 -[4-(4-bromophénoxy)phényl]-2-(phénoxyméthyl)-1H-imidazole ;
4-[4-(1H-imidazol-1-yl)phényl]-2-(phénoxyméthyl)-1H-imidazole;
4-[4-(4-méthoxyphénoxy)phényl]-2-(phénoxyméthyl)-1H-imidazole ;
4-(4-hexylphényl)-2-(phénoxyméthyl)-1H-imidazole ;
4-(4-butoxyphényl)-2-(phénoxyméthyl)-1H-imidazole ;
4-[4-(4-nitrophénoxy)phényl]-2-(phénoxyméthyl)-1H-imidazole ;
4-(2-{4-[2-(phénoxyméthyl)-1H-imidazol-4-yl]phénoxy}éthyl) morpholine ;
chlorhydrate de 1-(2-{4-[2-(phénoxyméthyl)-1H-imidazol-4-yl]phénoxy} éthyl) pipéridine ;
chlorhydrate de N,N-diméthyl-N-(2-{4-[2-(phénoxyméthyl)-1H-imidazol-4-yl] phénoxy} éthyl) amine ;
4-[4-(2-méthoxyéthoxy)phényl]-2-(phénoxyméthyl)-1H-imidazole ;
2-(phénoxyméthyl)-4-[4-(4,4,4-trifluorobutoxy)phényl]-1H-imidazole ;
4-[4-(4-fluorophénoxy)phényl]-5-méthyl-2-(phénoxyméthyl)-1H-imidazole ;
4-fluoro-N-{4-[2-(phénoxyméthyl)-1H-imidazol-4-yl]phényl} benzamide ;
4-{4-[2-(phénoxyméthyl)-1H-imidazol-4-yl]phénoxy}benzonitrile ;
4-[2-(phénoxyméthyl)-1H-imidazol-4-yl] benzoate d'éthyle ;
4- {4-[2-(phénoxyméthyl)-1H-imidazol-4-yl]phénoxy}benzoate d'éthyle ;
4-{4-[4-(méthylthio)phénoxy]phényl}-2-(phénoxyméthyl)-1H-imidazole ;
4-{4-[4-(méthylsulfonyl)phénoxy]phényl}-2-(phénoxyméthyl)-1H-imidazole ;
chlorhydrate de 4-{4-[2-(phénoxyméthyl)-1H-imidazol-4-yl] phénoxy}aniline ;
trifluoroacétate de {4-[2-(phénoxyméthyl)-1H-imidazol-4-yl]phényl}phényl méthanone ;
trifluoroacétate de N-(4-fluorophényl)-4-[2-(phénoxyméthyl)-1H-imidazol-4-yl] benzamide ;
4-[4-(3-nitrophénoxy)phényl]-2-(phénoxyméthyl)-1H-imidazole ;
chlorhydrate de 3-{4-[2-(phénoxyméthyl)-1H-imidazol-4-yl]phénoxy}aniline ;
4-{4-[4-(benzyloxy)phénoxy]phényl}-2-(phénoxyméthyl)-1H-imidazole ;
4-{4-[2-(phénoxyméthyl)-1H-imidazol-4-yl]phénoxy}phénol ;
4-[4-(3-fluorophénoxy)phényl]-2-(phénoxymethyl)-1H-imidazole ;
N-(4-{4-[2-(phénoxyméthyl)-1H-imidazol-4-yl]phénoxy}phényl) acétamide ;
trifluoroacétate de 2-nitro-4-{4-[2-(phénoxyméthyl)-1H-imidazol-4-yl]phénoxy} aniline;
N-méthyl-N-(4-{4-[2-(phénoxyméthyl)-1H-imidazol-4-yl]phénoxy}phényl) amine ;
3-{4-[2-(phénoxyméthyl)-1H-imidazol-4-yl]phénoxy}benzonitrile ;
4-[4-(2-nitrophénoxy)phényl]-2-(phénoxyméthyl)-1H-imidazole;
chlorhydrate de 2-{4-[2-(phénoxyméthyl)-1H-imidazol-4-yl]phénoxy}aniline;
chlorhydrate de 1-(4-{4-[2-(phénoxyméthyl)-1H-imidazol-4-yl]phénoxy}phényl) méthanamine ;
chlorhydrate de 1-(3-{4-[2-(phénoxyméthyl)-1H-imidazol-4-yl]phénoxy}phényl) méthanamine ;
4-[4-(3-bromophénoxy)phényl]-2-(phénoxyméthyl)-1H-imidazole ;
chlorhydrate de 2-fluoro-4-{4-[2-(phénoxyméthyl)-1H-imidazol-4-yl]phénoxy} aniline ;
4-[4-(3-chlorophénoxy)phényl]-2-(phénoxyméthyl)-1H-imidazole;
4-[4-(3,5-difluorophénoxy)phényl]-2-(phénoxyméthyl)-1H-imidazole ;
4-(4-benzylphényl)-2-(phénoxyméthyl)-1H-imidazole ;
4-[4-(3-méthylphénoxy)phényl]-2-(phénoxyméthyl)-1H-imidazole ;
chlorhydrate de 4-[4-(2-chlorophénoxy)phényl]-2-(phénoxyméthyl)-1H-imidazole ;
4-[4-(2-fluorophénoxy)phényl]-2-(phénoxyméthyl)-1H-imidazole ;
4-[4-(3,4-difluorophénoxy)phényl]-2-(phénoxyméthyl)-1H-imidazole ;
chlorhydrate de N¹-(4-{4-[2-(phénoxyméthyl)-1H-imidazol-4-yl]phénoxy}phényl) glycinamide ;
4-[4-(2,5-difluorophénoxy)phényl]-2-(phénoxyméthyl)-1H-imidazole ;
4-[4-(2,4-difluorophénoxy)phényl]-2-(phénoxyméthyl)-1H-imidazole ;
4-[4-(2,3-difluorophénoxy)phényl]-2-(phénoxyméthyl)-1H-imidazole ;
4-[4-(2,6-difluorophénoxy)phényl]-2-(phénoxyméthyl)-1H-imidazole.

17. Procédé de préparation d'un composé de fonnule (I) selon l'une des revendications précédentes **caractérisé en ce que** l'on fait réagir un composé de formule 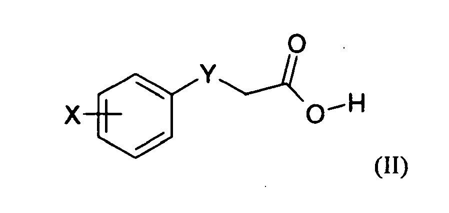 dans laquelle X et Y ont la signification indiquée ci-dessus, avec une base pour former le composé (II) sous une forme salifiée, puis avec l'α-halogéno-cétone de formule 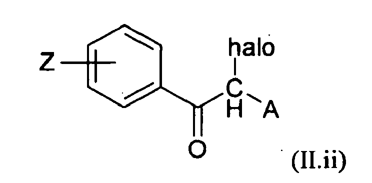 dans laquelle Z et A ont la signification indiquée ci-dessus, dans un solvant inerte,
puis le céto-ester ainsi obtenu est cyclisé en présence d'un sel d'ammonium pour donner le composé de formule (I).

18. Procédé de préparation d'un composé de formule (I) selon l'une des revendications 1 à 16, **caractérisé en ce que** l'on condense au reflux dans un solvant inerte polaire, un composé de formule (II-iii) 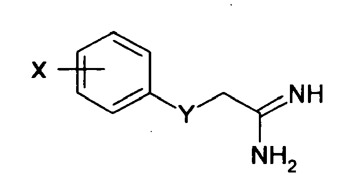 dans laquelle X et Y ont la signification indiquée à l'une des revendications précédentes, et l'α-halogéno-cétone de formule générale (II-ii) 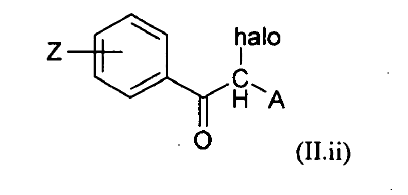 dans lequel Z et A sont tels que définis à l'une des revendications précédentes.

19. Composition pharmaceutique contenant, à titre de principe actif, au moins un composé selon l'une des revendications 1 à 16, en association avec un support pharmaceutiquement acceptable.

20. Utilisation d'un composé selon l'une des revendications 1 à 16, pour la préparation d'un médicament anti-tumoral.

21. Utilisation d'un composé selon l'une des revendications 1 à 16, pour la préparation d'un médicament destiné à inhiber la polymérisation de la tubuline.

22. Utilisation d'un composé de formule (I') 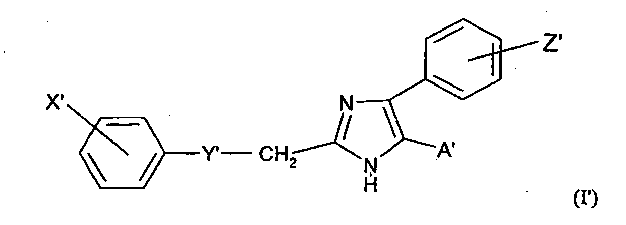 sous forme racémique, d'énantiomère ou toutes combinaisons de ces formes, et dans laquelle
X' représente un ou plusieurs substituants identiques ou différents choisis parmi H et halo ;
Y' représente -O- ou -S- ;
A' représente H ou (C₁-C₆)alkyle ;
Z' représente un ou plusieurs substituants identiques ou différents choisis parmi:
. (C₁-C₆)alkyle éventuellement substitué par un ou plusieurs radicaux halo identiques ou différents ;
. aryle éventuellement substitué par un ou plusieurs radicaux identiques ou différents choisis parmi : halo, nitro, cyano, hydroxy, (C₁-C₆)alkyle éventuellement substitué par un ou plusieurs radicaux halo identiques ou différents, -(CH₂)ₙ-NR₃R₄, (C₁-C₆)alkyl-sulfonyle, (C₁-C₆)alkyl-thio, (C₁-C₆)alkoxy éventuellement substitué par un ou plusieurs radicaux halo identiques ou différents, (C₁-C₆)alkoxy-carbonyle, phosphate, sulfate, glycoside et -NH-C(O)-CH(R_{A})-NR₅R₆;
. aryl-(C₁-C₆)alkyle ;
. hétéroaryle ;
. -Z₁-Z'₁;
. -NR_{N}-C(O)-Z'₂; ou
. -Z₂-Z'₂;
Z₁ représente -O- ,-C(O)-O-, -NR_{N}-C(O)- ou -C(O)-NR_{N}- ;
Z'₁ représente un radical (C₁-C₁₀)alkyle; aryle-(C₁-C₆)alkyle dont le radical aryle est éventuellement substitué par un ou plusieurs halo identiques ou différents ; ou (C₁-C₆)alkyle substitué par un ou plusieurs substituants choisis parmi : halo, (C₁-C₆)alkoxy, (C₁-C₆)alkylthio et -NR₁R₂ ;
R₁ et R₂ représentent, indépendamment, H ou (C₁-C₆)alkyle, ou bien forment ensemble avec l'atome d'azote sur lequel ils sont rattachés, un hétérocycloalkyle éventuellement substitué par (C₁-C₆)alkyle ;
Z₂ représente -O-, -S-, -SO₂- -C(O)-, -C(O)-NR_{N}- ou NR_{N}- ;
Z'₂ représente un radical aryle ou hétéroaryle, les radicaux aryle et hétéroaryle étant éventuellement substitués par un ou plusieurs radicaux identiques ou différents choisis parmi :
halo, nitro, cyano, hydroxy, (C₁-C₆)alkyle éventuellement substitué par un ou plusieurs radicaux halo identiques ou différents, (C₁-C₆)alkyl-thio, (C₁-C₆)alkyl-sulfonyle, (C₁-C₆)alkoxy éventuellement substitué par un ou plusieurs radicaux halo identiques ou différents, aryl-alkoxy, (C₁-C₆)alkoxy-carbonyle, phosphate, sulfate, glycoside, -(CH₂)ₙ-NR₃R₄ et -NH-C(O)-CH(R_{A})-NR₅R₆ ;
R₃ et R₄ représentent, indépendamment, H, (C₁-C₆)alkyle, (C₁-C₆)alkyl-carbonyle ou (C₁-C₆)alkyl-sulfonyle, ou bien R₃ et R₄ forment ensemble avec l'atome d'azote sur lequel ils sont rattachés, un hétéroaryle ou un hétérocycloalkyle éventuellement substitué par (C₁-C₆)alkyle ;
R₅ et R₆ représentent, indépendamment, H ou (C₁-C₆)alkyle ;
R_{A} représente le résidu associé à l'acide aminé de formule NH₂-CH(R_{A})-C(O)-OH ;
R_{N} représente l'hydrogène ou un radical (C₁-C₆)alkyle ;
n représente un entier de 0 à 3 ; ou un sel pharmaceutiquement acceptable de ces composés, pour la préparation d'un médicament anti-tumoral.

23. Utilisation d'un composé de formule (I') tel que défini à la revendication 22, pour la préparation d'un médicament destiné à inhiber la polymérisation de la tubuline.

## Claims

1. Compound of general formula (I) 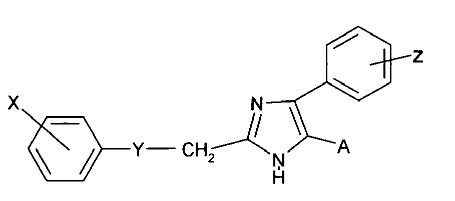 in racemic, enantiomeric form or any combinations of these forms and in which :
X represents one or more identical or different substituents chosen from H and halo ;
Y represents -O- or -S-;
A represents H or (C₁-C₆)alkyl ;
Z represents one or more identical or different substituents chosen from:
. (C₁-C₆)alkyl optionally substituted by one or more identical or different halo radicals;
. aryl optionally substituted by one or more identical or different radicals chosen from : halo, nitro, cyano, hydroxy, (C₁-C₆)alkyl optionally substituted by one or more identical or different halo radicals, -(CH₂)ₙ-NR₃R₄, (C₁-C₆)alkylsulphonyl, (C₁-C₆)alkyl-thio, -(C₁-C₆)alkoxy optionally substituted by one or more identical or different halo radicals, (C₁-C₆)alkoxy-carbonyl, phosphate, sulphate, glycoside and -NH-C(O)-CH(R_{A})-NR₅R₆ ;
. aryl-(C₁-C₆)alkyl ;
. heteroaryl;
. - Z₁-Z'₁;
. -NR_{N}-C(O)-Z'₂; or
. -Z₂-Z'₂;
Z₁ represents -O-, -C(O)-O-, -NR_{N}-C(O)- or -C(O)-NR_{N}- -;
Z'₁ represents a (C₁-C₁₀)alkyl radical; aryl-(C₁-C₆)alkyl the aryl radical of which is optionally substituted by one or more idendical or different halo radicals; or (C₁-C₆)alkyl substituted by one or more substituents chosen from halo, (C₁-C₆)alkoxy, (C₁-C₆)alkylthio and -NR₁R₂;
R₁ and R₂ represent, independently, H or (C₁-C₆)alkyl, or form together with the nitrogen atom to which they are attached, a heterocycloalkyl optionally substituted by (C₁-C₆)alkyl;
Z₂ represents -O-, -S-, -SO₂-, -C(O)-, -C(O)-NR_{N}- or -NR_{N}-;
Z'₂ represents an aryl or heteroaryl radical, the aryl and heteroaryl radicals being optionally substituted by one or more identical or different radicals chosen from :
halo, nitro, cyano, hydroxy, (C₁-C₆)alkyl optionally substituted by one or more identical or different halo radicals, (C₁-C₆)alkyl-thio, (C₁-C₆)alkyl-sulphonyl, -(C₁-C₆)alkoxy optionally substituted by one or more identical or different halo radicals, aryl-alkoxy, (C₁-C₆)alkoxy-carbonyl, phosphate, sulphate, glycoside, -(CH₂)ₙ-NR₃R₄ and -NH-C(O)-CH(R_{A})-NR₅R₆;
R₃ et R₄ represent, independently, H, (C₁-C₆)alkyl, (C₁-C₆)alkyl-carbonyl or (C₁-C₆)alkyl-sulphonyl, or R₃ and R₄ form together with the nitrogen atom to which they are attached, a heteroaryl or heterocycloalkyl optionally substituted by (C₁-C₆)alkyl;
R₅ and R₆ represent, independently, H or (C₁-C₆)alkyl;
R_{A} represents the residue associated with the amino acid of formula NH₂-CH(R_{A})-C(O)-OH;
R_{N} represents H or a (C₁-C₆)alkyl radical ;
n represents an integer from 0 to 3 ; or a pharmaceutically acceptable salt thereof ;
to the exclusion of compounds in which A represents the hydrogen atom and Z a -3-CF₃ radical.

2. Compound according to claim 1, **characterized in that**
X represents one or more identical or different substituents chosen from H and halo;
Y represents -O- or -S-;
A represents H or (C₁-C₆)alkyl;
Z represents one or more identical or different substituents chosen from:
. (C₁-C₆)alkyl optionally substituted by one or more identical or different halo radicals;
. aryl optionally substituted by one or more identical or different radicals chosen from : halo, nitro, cyano, hydroxy, (C₁-C₆)alkyl optionally substituted by one or more identical or different halo radicals, and -(C₁-C₆)alkoxy optionally substituted by one or more identical or different halo radicals ;
. heteroaryl ;
. -Z₁-Z'₁;
. -NH-C(O)-Z'₂ ; or
. - Z₂-Z'₂;
Z₁ represents -O-, -NH-C(O)- or -C(O)-NH-;
Z'₁ represents a (C₄-C₁₀)alkyl radical; aryl-(C₁-C₆)alkyl the aryl radical of which is optionally substituted by one or more identical or different halo radicals; or (C₁-C₆)alkyl substituted by one or more substituents chosen from halo, (C₁-C₆)alkoxy, (C₁-C₆)alkylthio and -NR₁R₂;
R₁ and R₂ represent, independently, H or (C₁-C₆)alkyl, or form together with the nitrogen atom to which they are attached, a heterocycloalkyl optionally substituted by (C₁-C₆)alkyl;
Z₂ represents -O-, -S-, -SO₂-, -C(O)- or -C(O)-NH-;
Z'₂ represents an aryl optionally substituted by one or more identical or different radicals chosen from : halo, nitro, cyano, hydroxy, (C₁-C₆)alkyl optionally substituted by one or more identical or different halo radicals, and -(C₁-C₆)alkoxy optionally substituted by one or more identical or different halo radicals ; or a pharmaceutically acceptable salt thereof.

3. Compound according to claim 1 or 2, **characterized in that** A represents H and Y represents -O-; or a pharmaceutically acceptable salt thereof.

4. Compound according to one of claim 1 to 3, **characterized in that** X represents H ; or a pharmaceutically acceptable salt thereof.

5. Compound according to claim 4, **characterized in that**
Z represents one or more identical or different substituents, in meta and/or para position and chosen from heteroaryl and -Z₂-Z'₂;
Z₂ represents -O-, -S-, -SO₂-, -C(O)- or -C(O)-NH-;
Z'₂ represents one of the phenyl or naphthyl radicals optionally substituted by one or more identical or different radicals chosen from halo, nitro, cyano, hydroxy, (C₁-C₆)alkyl optionally substituted by one or more identical or different halo radicals, and -(C₁-C₆)alkoxy optionally substituted by one or more identical or different halo radicals; or a pharmaceutically acceptable salt thereof.

6. Compound according to one of claims 1, 3 or 4, **characterized in that** Z represents
. a heteroaryl;
. -Z₁-Z'₁ in which
either Z₁ represents -O-, -NR_{N}-C(O)- or -C(O)-NR_{N}- and Z'₁ represents the benzyl radical;
or Z₁ represents -O-, -C(O)-O-, -NR_{N}-C(O)- or -C(O)-NR_{N}- and Z'₁ represents a (C₁-C₆)alkyl radical substituted by one or more substituents chosen from: halo, (C₁-C₆)alkoxy, (C₁-C₆)alkylthio and -NR₁R₂;
R₁ and R₂ represent, independently, H or (C₁-C₆)alkyl, or form together with the nitrogen atom to which they are attached, a heterocycloalkyl;
. -Z₂-Z'₂ in which
Z₂ represents -O-, -S-, -SO₂-, -C(O)-, -C(O)-NR_{N}- or NR_{N}-;
Z'₂ represents a phenyl radical or phenyl substituted by one or more identical or different radicals chosen from:
halo, nitro, cyano, hydroxy, (C₁-C₆)alkyl optionally substituted by one or more identical or different halo radicals, (C₁-C₆)alkyl-thio, (C₁-C₆)alkyl-sulphonyl, (C₁-C₆)alkoxy optionally substituted by one or more identical or different halo radicals, aryl-alkoxy, (C₁-C₆)alkoxycarbonyl, -(CH₂)ₙ-NR₃R₄ and -NH-C(O)-CH(R_{A})-NR₅R₆;
R₃ and R₄ represent, independently, H, (C₁-C₆)alkyl or (C₁-C₆)alkylcarbonyl ;
R₅ and R₆ represent, independently, H or (C₁-C₆)alkyl;
R_{A} represents the residue associated with the amino acid of formula NH₂-CH(R_{A})-C(O)-OH;
R_{N} represents hydrogen or a (C₁-C₆)alkyl radical; or a pharmaceutically acceptable salt thereof.

7. Compound according to one of claims 1, 3 or 4, **characterized in that** Z represents one or more substituents, identical or different, of formula -Z₂-Z'₂ ; or a pharmaceutically acceptable salt thereof.

8. Compound according to claim 7, **characterized in that** Z is in meta and/or para position; or a pharmaceutically acceptable salt thereof.

9. Compound according to one of claims 7 to 8, **characterized in that** Z₂ represents -O-, -S-, -SO₂- or -C(O)-; or a pharmaceutically acceptable salt thereof.

10. Compound according to claim 9, **characterized in that** Z₂ represents -O- ; or a pharmaceutically acceptable salt thereof.

11. Compound according to one of claims 7 to 8, **characterized in that** Z₂ represents -NR_{N}-; or a pharmaceutically acceptable salt thereof.

12. Compound according to one of claims 7 to 11, **characterized in that**
Z'₂ represents a phenyl radical or phenyl substituted by one or more identical or different radicals chosen from:
halo, nitro, cyano, hydroxy, (C₁-C₆)alkyl optionally substituted by one or more identical or different halo radicals, (C₁-C₆)alkyl-thio, (C₁-C₆)alkylsulphonyl, (C₁-C₆)alkoxy optionally substituted by one or more identical or different halo radicals, benzyl-alkoxy, (C₁-C₆)alkoxy-carbonyl, phosphate, -(CH₂)ₙ-NR₃R₄ and -NH-C(O)-CH(R_{A})-NR₅R₆ ;
R₃ and R₄ represent, independently, H, (C₁-C₆)alkyl, (C₁-C₆)alkyl-carbonyl or (C₁-C₆)alkyl-sulphonyl ;
R_{N} represents hydrogen or a (C₁-C₆)alkyl radical;
R₅ and R₆ represent, independently, H or (C₁-C₆)alkyle;
R_{A} represents the residue associated with the amino acid of formula NH₂-CH(R_{A})-C(O)-OH ; or a pharmaceutically acceptable salt thereof.

13. Compound according to one of claims 7 to 12 **characterized in that**
Z'₂ represents phenyl substituted by one or more identical or different radicals chosen from: halo, nitro, cyano, hydroxy, (C₁-C₆)alkyl-sulphonyl, (C₁-C₆)alkoxy, -(CH₂)ₙ-NR₃R₄ and -NH-C(O)-CH(R_{A})-NR₅R₆;
R₃ and R₄ represent, independently, H, (C₁-C₆)alkyl or (C₁-C₆)alkyl-carbonyl;
R₅ and R₆ represent, independently, H or (C₁-C₆)alkyl; or a pharmaceutically acceptable salt thereof.

14. Compound according to claim 13 **characterized in that**
Z'₂ represents phenyl substituted by at least two identical or different radicals chosen from: fluoro, nitro, cyano, hydroxy, (C₁-C₆)alkyl-sulphonyl, (C₁-C₆)alkoxy, -NH₂ and NH-C(O)-CH(R_{A})-NR₅R₆; R₅ and R₆ represent, independently, H or (C₁-C₆)alkyl; or a pharmaceutically acceptable salt thereof.

15. Compound according to one of claims 7 to 11, **characterized in that**
Z'₂ represents the pyridinyl, pyrimidinyl, pyrazinyl, triazolyl, furyl, thienyl, purinyl, triazinyl, pyrrazolo-pyrimidinyl, quinoxalinyl or indolyl radical,
each of these radicals being optionally substituted by one or more identical or different radicals chosen from: halo, nitro, cyano, hydroxy, (C₁-C₆)alkyl and - NH₂; or a pharmaceutically acceptable salt thereof.

16. Compound according to claim 1 **characterized in that** it corresponds to one of the following formulae:
4-[4-(4-fluorophenoxy)phenyl]-2-(phenoxymethyl)-1 H-imidazole;
4-(1,1'-biphenyl-4-yl)-2-[(phenylthio)methyl]-1H-imidazole;
4-(1,1'-biphenyl-4-yl)-2-(phenoxymethyl)-1H-imidazole;
4-[4-(4-fluorophenoxy)phenyl]-2-[(phenylthio)methyl]-1H-imidazole;
2-[(4-fluorophenoxy)methyl]-4-[4-(4-fluorophenoxy)phenyl]-1H-imidazole;
2-(phenoxymethyl)-4-[4-(phenylthio)phenyl]-1H-imidazole;
2-(phenoxymethyl)-4-[4-(phenylsulphonyl)phenyl]-1H-imidazole;
4-{4-[(2-fluorobenzyl)oxy]phenyl}-2-(phenoxymethyl)-1H-imidazole;
2-(phenoxymethyl)-4-(4-phenoxyphenyl)-1H-imidazole trifluoroacetate;
4-[4-(4-bromophenoxy)phenyl]-2-(phenoxymethyl)-1H-imidazole trifluoroacetate;
4-[4-(1H-imidazol-1-yl)phenyl]-2-(phenoxymethyl)-1H-imidazole;
4-[4-(4-methoxyphenoxy)phenyl]-2-(phenoxymethyl)-1H-imidazole;
4-(4-hexylphenyl)-2-(phenoxymethyl)-1H-imidazole;
4-(4-butoxyphenyl)-2-(phenoxymethyl)-1H-imidazole;
4-[4-(4-nitrophenoxy)phenyl]-2-(phenoxymethyl)-1H-imidazole;
4-(2-{4-[2-(phenoxymethyl)-1H-imidazol-4-yl]phenoxy}ethyl) morpholine;
1-(2-{4-[2-(phenoxymethyl)-1H-imidazol-4-yl]phenoxy} ethyl) piperidine hydrochloride;
N,N-dimethyl-N-(2-{4-[2-(phenoxymethyl)-1H-imidazol-4-yl] phenoxy}ethyl) amine hydrochloride;
4-[4-(2-methoxyethoxy)phenyl]-2-(phenoxymethyl)-1H-imidazole;
2-(phenoxymethyl)-4-[4-(4,4,4-trifluorobutoxy)phenyl]-1H-imidazole;
4-[4-(4-fluorophenoxy)phenyl]-5-methyl-2-(phenoxymethyl)-1H-imidazole;
4-fluoro-N-{4-[2-(phenoxymethyl)-1H-imidazol-4-yl]phenyl} benzamide;
4-{4-[2-(phenoxymethyl)-1H-imidazol-4-yl]phenoxy}benzonitrile;
ethyl 4-[2-(phenoxymethyl)-1H-imidazol-4-yl] benzoate;
ethyl 4-{4-[2-(phenoxymethyl)-1H-imidazol-4-yl]phenoxy}benzoate;
4-{4-[4-(methylthio)phenoxy]phenyl}-2-(phenoxymethyl)-1H-imidazole;
4-{4-[4-(methylsulphonyl)phenoxy]phenyl}-2-(phenoxymethyl)-1H-imidazole;
4-{4-[2-(phenoxymethyl)-1H-imidazol-4-yl] phenoxy}aniline hydrochloride;
{4-[2-(phenoxymethyl)-1H-imidazol-4-yl]phenyl}phenyl methanone trifluoroacetate;
N-(4-fluorophenyl)-4-[2-(phenoxymethyl)-1H-imidazol-4-yl] benzamide trifluoroacetate;
4-[4-(3-nitrophenoxy)phenyl]-2-(phenoxymethyl)-1H-imidazole;
3-{4-[2-(phenoxymethyl)-1H-imidazol-4-yl]phenoxy}aniline hydrochloride;
4- {4-[4-(benzyloxy)phenoxy]phenyl}-2-(phenoxymethyl)-1H-imidazole;
4-{4-[2-(phenoxymethyl)-1H-imidazol-4-yl]phenoxy}phenol;
4-[4-(3-fluorophenoxy)phenyl]-2-(phenoxymethyl)-1H-imidazole;
N-(4-{4-[2-(phenoxymethyl)-1H-imidazol-4-yl]phenoxy}phenyl) acetamide;
2-nitro-4-{4-[2-(phenoxymethyl)-1H-imidazol-4-yl]phenoxy} aniline trifluoroacetate;
N-methyl-N-(4-{4-[2-(phenoxymethyl)-1H-imidazol-4-yl]phenoxy}phenyl) amine;
3-{4-[2-(phenoxymethyl)-1H-imidazol-4-yl]phenoxy}benzonitrile;
4-[4-(2-nitrophenoxy)phenyl]-2-(phenoxymethyl)-1H-imidazole;
2-{4-[2-(phenoxymethyl)-1H-imidazol-4-yl]phenoxy}aniline hydrochloride;
1-(4-{4-[2-(phenoxymethyl)-1H-imidazol-4-yl]phenoxy}phenyl) methanamine hydrochloride;
1-(3-{4-[2-(phenoxymethyl)-1H-imidazol-4-yl]phenoxy}phenyl) methanamine hydrochloride;
4-[4-(3-bromophenoxy)phenyl]-2-(phenoxymethyl)-1H-imidazole;
2-fluoro-4-{4-[2-(phenoxymethyl)-1H-imidazol-4-yl]phenoxy} aniline hydrochloride;
4-[4-(3-chlorophenoxy)phenyl]-2-(phenoxymethyl)-1H-imidazole;
4-[4-(3,5-difluorophenoxy)phenyl]-2-(phenoxymethyl)-1H-imidazole;
4-(4-benzylphenyl)-2-(phenoxymethyl)-1H-imidazole;
4-[4-(3-methylphenoxy)phenyl]-2-(phenoxymethyl)-1H-imidazole;
4-[4-(2-chlorophenoxy)phenyl]-2-(phenoxymethyl)-1H-imidazole hydrochloride;
4-[4-(2-fluorophenoxy)phenyl]-2-(phenoxymethyl)-1H-imidazole;
4-[4-(3,4-difluorophenoxy)phenyl]-2-(phenoxymethyl)-1H-imidazole;
N¹-(4-{4-[2-(phenoxymethyl)-1H-imidazol-4-yl]phenoxy}phenyl) glycinamide hydrochloride;
4-[4-(2,5-difluorophenoxy)phenyl]-2-(phenoxymethyl)-1H-imidazole;
4-[4-(2,4-difluorophenoxy)phenyl]-2-(phenoxymethyl)-1H-imidazole;
4-[4-(2,3-difluorophenoxy)phenyl]-2-(phenoxymethyl)-1H-imidazole;
4-[4-(2,6-difluorophenoxy)phenyl]-2-(phenoxymethyl)-1H-imidazole.

17. Process for the preparation of a compound of formula (I) according to one of the preceding claims **characterized in that** a compound of formula 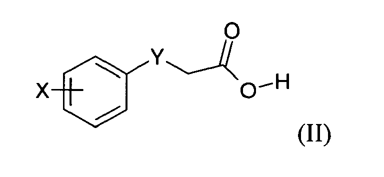 in which X and Y have the meaning indicated above, is reacted with a base in order to form compound (II) in a salified form, then with the α-halogeno-ketone of formula 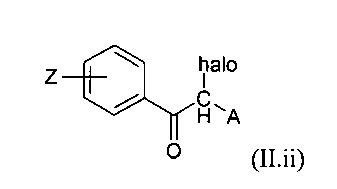 in which Z and A have the meaning indicated above, in an inert solvent,
then the keto-ester thus obtained is cyclized in the presence of an ammonium salt in order to produce the compound of formula (I).

18. Process for the preparation of a compound of formula (I) according to one of the claims 1 to 16 **characterized in that** a compound of formula (II-iii) 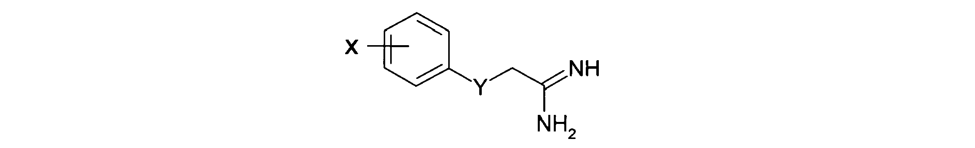 in which X and Y have the meaning indicated in one of the preceding claims, and the α-halogeno-ketone of general formula (II-ii) 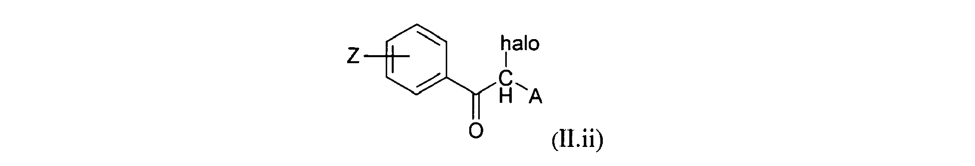 in which Z and A are as defined in one of the preceding claims, are condensed under reflux in a polar inert solvent.

19. Pharmaceutical composition containing, as active ingredient, at least one compound according to one of claims 1 to 16, in combination with a pharmaceutically acceptable support.

20. Use of a compound according to one of claims 1 to 16, for the preparation of an anti-tumorous medicament.

21. Use of a compound according to one of claims 1 to 16, for the preparation of a medicament intended to inhibit tubulin polymerization.

22. Use of a compound of formula (I') 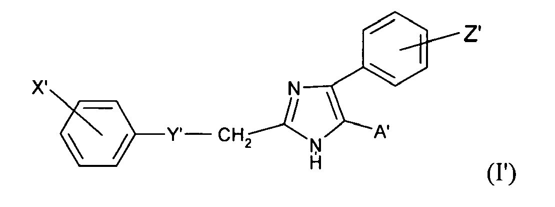 in racemic, enantiomeric form or any combinations of these forms, and in which
X' represents one or more identical or different substituents chosen from H and halo;
Y' represents -O- or -S-;
A' represents H or (C₁-C₆)alkyl;
Z' represents one or more identical or different substituents chosen from:
. (C₁-C₆)alkyl optionally substituted by one or more identical or different halo radicals;
. aryl optionally substituted by one or more identical or different radicals chosen from: halo, nitro, cyano, hydroxy, (C₁-C₆)alkyl optionally substituted by one or more identical or different halo radicals, -(CH₂)ₙ-NR₃R₄, (C₁-C₆)alkylsulphonyl, (C₁-C₆)alkyl-thio, (C₁-C₆)alkoxy optionally substituted by one or more identical or different halo radicals, (C₁-C₆)alkoxy-carbonyl, phosphate, sulphate, glycoside and -NH-C(O)-CH(R_{A})-NR₅R₆;
. aryl-(C₁-C₆)alkyl;
. heteroaryl;
. - Z₁-Z'₁;
. -NR_{N}-C(O)-Z'₂; or
. -Z₂-Z'₂;
Z₁ represents -O-, -C(O)-O-, -NR_{N}-C(O)- or -C(O)-NR_{N}-;
Z'₁ represents a (C₁-C₁₀)alkyl radical; aryl-(C₁-C₆)alkyl the aryl radical of which is optionally substituted by one or more identical or different halo radicals; or (C₁-C₆)alkyl substituted by one or more substituents chosen from: halo, (C₁-C₆)alkoxy, (C₁-C₆)alkylthio and -NR₁R₂;
R₁ and R₂ represent, independently, H or (C₁-C₆)alkyl, or form together with the nitrogen atom to which they are attached, a heterocycloalkyl optionally substituted by (C₁-C₆)alkyl;
Z₂ represents -O-, -S-, -SO₂-, -C(O)-, -C(O)-NR_{N}- or NR_{N}-;
Z'₂ represents an aryl or heteroaryl radical, the aryl and heteroaryl radicals being optionally substituted by one or more identical or different radicals chosen from:
halo, nitro, cyano, hydroxy, (C₁-C₆)alkyl optionally substituted by one or more identical or different halo radicals, (C₁-C₆)alkyl-thio, (C₁-C₆)alkyl-sulphonyl, (C₁-C₆)alkoxy optionally substituted by one or more identical or different halo radicals, aryl-alkoxy, (C₁-C₆)alkoxy-carbonyl, phosphate, sulphate, glycoside, - (CH2)ₙ-NR₃R₄ and -NH-C(O)-CH(R_{A})-NR₅R₆;
R₃ and R₄ represent, independently, H, (C₁-C₆)alkyl, (C₁-C₆)alkyl-carbonyl or (C₁-C₆)alkyl-sulphonyl, or R₃ and R₄ form together with the nitrogen atom to which they are attached, a heteroaryl or a heterocycloalkyl optionally substituted by (C₁-C₆)alkyl;
R₅ and R₆ represent, independently, H or (C₁-C₆)alkyl;
R_{A} represents the residue associated with the amino acid of formula NH₂-CH(R_{A})-C(O)-OH;
R_{N} represents hydrogen or a (C₁-C₆)alkyl radical;
n represents an integer from 0 to 3 ; or a pharmaceutically acceptable salt of these compounds, for the preparation of an anti-tumorous medicament.

23. Use of a compound of formula (I') as defined in claim 22, for the preparation of a medicament intended to inhibit tubulin polymerization.

## Patentansprüche

1. Verbindung der allgemeinen Formel (I) 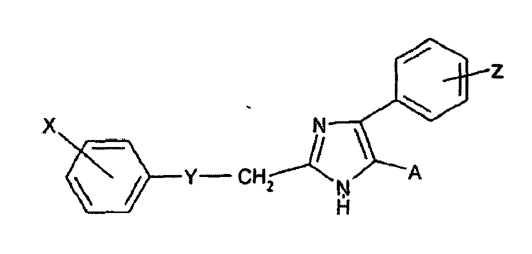 in racemischer Form, Enantiomerenform oder allen Kombinationen dieser Formen, in der:
X einen oder mehrere gleiche oder verschiedene Substituenten darstellt, die aus H und Halogen ausgewählt sind;
Y -O- oder -S- darstellt;
A H oder (C₁-C₆) -Alkyl darstellt;
Z einen oder mehrere gleiche oder verschiedene Substituenten darstellt, die ausgewählt sind aus:
. (C₁-C₆)-Alkyl, das gegebenenfalls mit einem oder mehreren gleichen oder verschiedenen Halogenresten substituiert ist;
. Aryl, das gegebenenfalls mit einem oder mehreren gleichen oder verschiedenen Resten substituiert ist, die ausgewählt sind aus: Halogen, Nitro, Cyano, Hydroxy, (C₁-C₆)-Alkyl, das gegebenenfalls mit einem oder mehreren gleichen oder verschiedenen Halogenresten substituiert ist, -(CH₂)ₙ-NR₃R₄, (C₁-C₆) -Alkylsulfonyl, (C₁-C₆)-Alkylthio, (C₁-C₆)-Alkoxy, das gegebenenfalls mit einem oder mehreren gleichen oder verschiedenen Halogenresten substituiert ist, (C₁-C₆)-Alkoxycarbonyl, Phosphat, Sulfat, Glycosid und -NH-C(O)-CH(R_{A})-NR₅R₆;
. Aryl-(C₁-C₆)-Alkyl;
. Heteroaryl;
. -Z₁-Z'₁;
. -NR_{N}-C(O)-Z'₂; oder
. -Z₂-Z'₂;
Z₁ -O-, -C(O)-O-, -NR_{N}-C(O)- oder -C(O)-NR_{N}- darstellt;
Z'₁ darstellt: einen Rest (C₁-C₁₀)-Alkyl; Aryl-(C₁-C₆)-Alkyl, dessen Arylrest gegebenenfalls mit einem oder mehreren gleichen oder verschiedenen Halogenresten substituiert ist; oder (C₁-C₆)-Alkyl, das durch einen oder mehrere Substituenten substituiert ist, die ausgewählt sind aus: Halogen, (C₁-C₆)-Alkoxy, (C₁-C₆)-Alkylthio und -NR₁R₂;
R₁ und R₂ unabhängig voneinander H oder (C₁-C₆)-Alkyl darstellen oder zusammen mit dem Stickstoffatom, an das sie gebunden sind, ein gegebenenfalls mit (C₁-C₆)-Alkyl substituiertes Heterocycloalkyl bilden;
Z₂ -O-, -S-, -SO₂-, -C(O)-, -C(O)-NR_{N}- oder -NR_{N}- darstellt;
Z'₂ einen Aryl- oder Heteroarylrest darstellt, wobei die Aryl- und Heteroarylreste gegebenenfalls mit einem oder mehreren gleichen oder verschiedenen Resten substituiert sind, die ausgewählt sind aus:
Halogen, Nitro, Cyano, Hydroxy, (C₁-C₆)-Alkyl, das gegebenenfalls mit einem oder mehreren gleichen oder verschiedenen Halogenresten substituiert ist, (C₁-C₆) -Alkylthio, (C₁-C₆) -Alkylsulfonyl, (C₁-C₆)-Alkoxy, das gegebenenfalls mit einem oder mehreren gleichen oder verschiedenen Halogenresten substituiert ist, Arylalkoxy, (C₁-C₆)-Alkoxycarbonyl, Phosphat, Sulfat, Glycosid, -(CH₂)ₙ-NR₃R₄ und -NH-C (O) -CH (R_{A}) -NR₅R₆;
R₃ und R₄ unabhängig voneinander H, (C₁-C₆)-Alkyl, (C₁-C₆)-Alkylcarbonyl oder (C₁-C₆)-Alkylsulfonyl darstellen oder R₃ und R₄ zusammen mit dem Stickstoffatom, an das sie gebunden sind, ein Heteroaryl oder ein Heterocycloalkyl bilden, das gegebenenfalls mit (C₁-C₆)-Alkyl substituiert ist;
R₅ und R₆ unabhängig voneinander H oder (C₁-C₆) -Alkyl darstellen;
R_{A} den mit der Aminosäure der Formel NH₂-CH(R_{A})-C(O)-OH assoziierten Rest darstellt;
R_{N} Wasserstoff oder einen (C₁-C₆)-Alkylrest darstellt;
n eine ganze Zahl von 0 bis 3 darstellt; oder ein pharmazeutisch annehmbares Salz dieser Verbindung
mit Ausnahme der Verbindungen, in denen A ein Wasserstoffatom und Z den Rest -3-CF₃ darstellt.

2. Verbindung nach Anspruch 1, **dadurch gekennzeichnet, dass**
X einen oder mehrere gleiche oder verschiedene Substituenten darstellt, die aus H und Halogen ausgewählt sind;
Y -O- oder -S- darstellt;
A H oder (C₁-C₆)-Alkyl darstellt;
Z einen oder mehrere gleiche oder verschiedene Substituenten darstellt, die ausgewählt sind aus:
. (C₁-C₆)-Alkyl, das gegebenenfalls mit einem oder mehreren gleichen oder verschiedenen Halogenresten substituiert ist;
. Aryl, das gegebenenfalls mit einem oder mehreren gleichen oder verschiedenen Resten substituiert ist, die ausgewählt sind, aus: Halogen, Nitro, Cyano, Hydroxy, (C₁-C₆)-Alkyl, das gegebenenfalls mit einem oder mehreren gleichen oder verschiedenen Halogenresten substituiert ist, und (C₁-C₆)-Alkoxy, das gegebenenfalls mit einem oder mehreren gleichen oder verschiedenen Halogenresten substituiert ist;
. Heteroaryl;
. -Z₁-Z'₁;
. -NH-C(O)-Z'₂; oder
. Z₂-Z'₂;
Z₁ -O-, -NH-C(O)- oder -C(O)-NH- darstellt;
Z'₁ darstellt: einen Rest (C₄-C₁₀)-Alkyl, Aryl-(C₁-C₆)-Alkyl, dessen Arylrest gegebenenfalls mit einem oder mehreren gleichen oder verschiedenen Halogenresten substituiert ist; oder (C₁-C₆)-Alkyl, das mit einem oder mehreren Substituenten substituiert ist, die aus Halogen, (C₁-C₆) -Alkoxy, (C₁-C₆) -Alkylthio und -NR₁R₂ ausgewählt sind;
R₁ und R₂ unabhängig voneinander H oder (C₁-C₆)-Alkyl darstellen oder zusammen mit dem Stickstoffatom, an das sie gebunden sind, ein gegebenenfalls mit (C₁-C₆)-Alkyl substituiertes Heterocycloalkyl bilden;
Z₂ -O-, -S-, -SO₂-, -C(O)- oder -C(O)-NH- darstellt;
Z'₂ einen Arylrest darstellt, der gegebenenfalls mit einem oder mehreren gleichen oder verschiedenen Resten substituiert ist, die ausgewählt sind aus: Halogen, Nitro, Cyano, Hydroxy, (C₁-C₆)-Alkyl, das gegebenenfalls mit einem oder mehreren gleichen oder verschiedenen Halogenresten substituiert ist, und (C₁-C₆)-Alkoxy, das gegebenenfalls mit einem oder mehreren gleichen oder verschiedenen Halogenresten substituiert ist; oder ein pharmazeutisch annehmbares Salz dieser Verbindung.

3. Verbindung nach einem der Ansprüche 1 bis 2, **dadurch gekennzeichnet, dass** A H darstellt und Y -O- darstellt; oder ein pharmazeutisch annehmbares Salz dieser Verbindung.

4. Verbindung nach einem der Ansprüche 1 bis 3, **dadurch gekennzeichnet, dass** X H darstellt; oder ein pharmazeutisch annehmbares Salz dieser Verbindung.

5. Verbindung nach Anspruch 4, **dadurch gekennzeichnet, dass**
Z einen oder mehrere gleiche oder verschiedene Substituenten in meta- und/oder para-Position darstellt, die aus Heteroaryl und -Z₂-Z'₂ ausgewählt sind;
Z₂ -O-, -S-, -SO₂-, -C(O)- oder -C(O)-NH- darstellt;
Z'₂ einen der Phenyl- oder Naphtylreste darstellt, die gegebenenfalls mit einem oder mehreren gleichen oder verschiedenen Resten substituiert sind, die ausgewählt sind aus Halogen, Nitro, Cyano, Hydroxy, (C₁-C₆)-Alkyl, das gegebenenfalls mit einem oder mehreren gleichen oder verschiedenen Halogenresten substituiert ist, und (C₁-C₆)-Alkoxy, das gegebenenfalls mit einem oder mehreren gleichen oder verschiedenen Halogenresten substituiert ist; oder ein pharmazeutisch annehmbares Salz dieser Verbindung.

6. Verbindung nach einem der Ansprüche 1, 3 oder 4, **dadurch gekennzeichnet, dass** Z darstellt:
. ein Heteroaryl;
. Z₁-Z'₁, in dem
entweder Z₁ -O-, -NR_{N}-C(O)- oder -C(O)-NR_{N}- darstellt und Z'₁ den Benzylrest darstellt;
oder Z₁ -O-, -C(O)-O-, -NR_{N}-C(O)- oder -C(O)-NR_{N}- darstellt und Z'₁ einen (C₁-C₆) - Alkylrest darstellt, der mit einem oder mehreren Substituenten substituiert ist, die ausgewählt sind aus: Halogen, (C₁-C₆)-Alkoxy, (C₁-C₆)-Alkylthio und -NR₁R₂;
R₁ und R₂ unabhängig voneinander H oder (C₁-C₆)-Alkyl darstellen oder zusammen mit dem Stickstoffatom, an das sie gebunden sind, ein Heterocycloalkyl bilden;
. Z₂-Z'₂, in dem
Z₂ -O-, -S-, -SO₂-, -C(O)-, -C(O)-NR_{N}- oder -NR_{N}- darstellt;
Z'₂ einen Phenylrest oder Phenyl darstellt, das mit einem oder mehreren gleichen oder verschiedenen Resten substituiert ist, die ausgewählt sind aus:
Halogen, Nitro, Cyano, Hydroxy, (C₁-C₆)-Alkyl, das gegebenenfalls mit einem oder mehreren gleichen oder verschiedenen Halogenresten substituiert ist, (C₁-C₆)-Alkylthio, (C₁-C₆)-Alkylsulfonyl, (C₁-C₆)-Alkoxy, das gegebenenfalls mit einem oder mehreren gleichen oder verschiedenen Halogenresten substituiert ist, Arylalkoxy, (C₁-C₆)-Alkoxycarbonyl, - (CH₂)ₙ-NR₃R₄ und -NH-C (O) -CH (R_{A}) -NR₅R₆;
R₃ und R₄ unabhängig voneinander H, (C₁-C₆)-Alkyl oder (C₁-C₆)-Alkylcarbonyl darstellen;
R₅ und R₆ unabhängig voneinander H oder (C₁-C₆)-Alkyl darstellen;
R_{A} den mit der Aminosäure der Formel NH₂-CH(R_{A})-C(O)-OH assoziierten Rest darstellt;
R_{N} Wasserstoff oder einen (C₁-C₆)-Alkylrest darstellt; oder ein pharmazeutisch annehmbares Salz dieser Verbindung.

7. Verbindung nach einem der Ansprüche 1, 3 oder 4, **dadurch gekennzeichnet, dass** Z einen oder mehrere gleiche oder verschiedene Substituenten der Formel -Z₂-Z'₂ darstellt; oder ein pharmazeutisch annehmbares Salz dieser Verbindung.

8. Verbindung nach Anspruch 7, **dadurch gekennzeichnet, dass** Z in meta- und/oder para-Position ist; oder ein pharmazeutisch annehmbares Salz dieser Verbindung.

9. Verbindung nach einem der Ansprüche 7 oder 8, **dadurch gekennzeichnet, dass** Z₂ -O-, -S-, -SO₂- oder -C(O)-darstellt; oder ein pharmazeutisch annehmbares Salz dieser Verbindung.

10. Verbindung nach Anspruch 9, **dadurch gekennzeichnet, dass** Z₂ -O- darstellt; oder ein pharmazeutisch annehmbares Salz dieser Verbindung.

11. Verbindung nach einem der Ansprüche 7 bis 8, **dadurch gekennzeichnet, dass** Z₂ -NR_{N}- darstellt; oder ein pharmazeutisch annehmbares Salz dieser Verbindung.

12. Verbindung nach einem der Ansprüche 7 bis 11, **dadurch gekennzeichnet, dass**
Z'₂ darstellt: Phenyl oder Phenyl, das mit einem oder mehreren gleichen oder verschiedenen Resten substituiert ist, die ausgewählt sind aus:
Halogen, Nitro, Cyano, Hydroxy, (C₁-C₆)-Alkyl, das gegebenenfalls mit einem oder mehreren gleichen oder verschiedenen Halogenresten substituiert ist, (C₁-C₆)-Alkylthio, (C₁-C₆) -Alkylsulfonyl, (C₁-C₆)-Alkoxy, das gegebenenfalls mit einem oder mehreren gleichen oder verschiedenen Halogenresten substituiert ist, Benzyloxy, (C₁-C₆)-Alkoxycarbonyl, Phosphat, -(CH₂)ₙ-NR₃R₄ und -NH-C (O) -CH(R_{A}) -NR₅R₆;
R₃ und R₄ unabhängig voneinander H, (C₁-C₆)-Alkyl, (C₁-C₆)-Alkylcarbonyl oder (C₁-C₆)-Alkylsulfonyl darstellen;
R_{N} Wasserstoff oder einen (C₁-C₆) -Alkylrest darstellt;
R₅ und R₆ unabhängig voneinander H oder (C₁-C₆)-Alkyl darstellen;
R_{A} den mit der Aminosäure der Formel NH₂-CH(R_{A})-C(O)-OH assoziierten Rest darstellt; oder ein pharmazeutisch annehmbares Salz dieser Verbindung.

13. Verbindung nach einem der Ansprüche 7 bis 12, **dadurch gekennzeichnet, dass**
Z'₂ Phenyl darstellt, das mit einem oder mehreren gleichen oder verschiedenen Resten substituiert ist, die ausgewählt sind aus: Halogen, Nitro, Cyano, Hydroxy, (C₁-C₆)-Alkylsulfonyl, (C₁-C₆)-Alkoxy, -(CH₂)ₙ-NR₃R₄ und -NH-C (O) -CH(R_{A})-NR₅R₆;
R₃ und R₄ unabhängig voneinander H, (C₁-C₆)-Alkyl oder (C₁-C₆)-Alkylcarbonyl darstellen;
R₅ und R₆ unabhängig voneinander H oder (C₁-C₆)-Alkyl darstellen; oder ein pharmazeutisch annehmbares Salz dieser Verbindung.

14. Verbindung nach Anspruch 13, **dadurch gekennzeichnet, dass**
Z'₂ Phenyl darstellt, das mit mindestens zwei gleichen oder verschiedenen Resten substituiert ist, die ausgewählt sind aus: Fluor, Nitro, Cyano, Hydroxy, (C₁-C₆)-Alkylsulfonyl, (C₁-C₆) -Alkoxy, -NH₂ und -NH-C(O)-CH(R_{A})-NR₅R₆; R₅ und R₆ unabhängig voneinander H oder (C₁-C₆)-Alkyl darstellen; oder ein pharmazeutisch annehmbares Salz dieser Verbindung.

15. Verbindung nach einem der Ansprüche 7 bis 11, **dadurch gekennzeichnet, dass**
Z'₂ den Rest Pyridinyl, Pyrimidinyl, Pyrazinyl, Triazolyl, Furyl, Thienyl, Purinyl, Triazinyl, Pyrrazolpyrimidinyl, Chinoxalinyl oder Indolyl darstellt;
wobei jeder der Reste gegebenenfalls mit einem oder mehreren gleichen oder verschiedenen Resten substituiert ist, die ausgewählt sind aus: Halogen, Nitro, Cyano, Hydroxy, (C₁-C₆)-Alkyl und -NH₂; oder ein pharmazeutisch annehmbares Salz dieser Verbindung.

16. Verbindung nach Anspruch 1, **dadurch gekennzeichnet, dass** sie einer der folgenden Formeln entspricht:
4-[4-(4-Fluorphenoxy)phenyl]-2-(phenoxymethyl)-1H-imidazol;
4-(1,1'-Biphenyl-4-yl)-2-[(phenylthio)methyl]-1H-imidazol;
4-(1,1'-Biphenyl-4-yl)-2-(phenoxymethyl)-1H-imidazol;
4-[4-(4-Fluorphenoxy)phenyl]-2-[(phenylthio)methyl]-1H-imidazol;
2-[(4-Fluorphenoxy)methyl]-4-[4-(4-fluorphenoxy)phenyl]-1H-imidazol;
2-(Phenoxymethyl)-4-[4-(phenylthio)phenyl]-1H-imidazol;
2-(Phenoxymethyl)-4-[4-(phenylsulfonyl)phenyl]-1H-imidazol;
4-{4-[(2-Fluorbenzyl)oxy]phenyl}-2-(phenoxymethyl)-1H-imidazol;
2-(Phenoxymethyl)-4-(4-phenoxyphenyl)-1H-imidazoltrifluoracetat;
4-[4-(4-Bromphenoxy)phenyl]-2-(phenoxymethyl)-1H-imidazoltrifluoracetat;
4-[4-(1H-Imidazol-1-yl)phenyl]-2-(phenoxymethyl)-1H-imidazol;
4-[4-(4-Methoxyphenoxy)phenyl]-2-(phenoxymethyl)-1H-imidazol;
4-(4-Hexylphenyl)-2-(phenoxymethyl)-1H-imidazol;
4-(4-Butoxyphenyl)-2-(phenoxymethyl)-1H-imidazol;
4-[4-(4-Nitrophenoxy)phenyl]-2-(phenoxymethyl)-1H-imidazol;
4-(2-{4-[2-(Phenoxymethyl)-1H-imidazol-4-yl]phenoxy}ethyl)morpholin;
1-(2-{4-[2-(Phenoxymethyl)-1H-imidazol-4-yl]phenoxy}ethyl)piperidinchlorhydrat;
N,N-Dimethyl-N-(2-{4-[2-(phenoxymethyl)-1H-imidazol-4-yl]phenoxy}ethyl)aminchlorhydrat;
4-[4-(2-Methoxyethoxy)phenyl]-2-(phenoxymethyl)-1H-imidazol;
2-(Phenoxymethyl)-4-[4-(4,4,4-trifluorbutoxy)phenyl]-1H-imidazol;
4-[4-(4-Fluorphenoxy)phenyl]-5-methyl-2-(phenoxymethyl)-1H-imidazol;
4-Fluor-N-{4-[2-(phenoxymethyl)-1H-imidazol-4-yl]phenyl}benzamid;
4-{4-[2-(Phenoxymethyl)-1H-imidazol-4-yl]phenoxy}benzonitril;
4-[2-(Phenoxymethyl)-1H-imidazol-4-yl]ethylbenzoat;
4-{4-[2-(Phenoxymethyl)-1H-imidazol-4-yl]phenoxy}ethylbenzoat;
4-{4-[4-(Methylthio)phenoxy]phenyl}-2-(phenoxymethyl)-1H-imidazol;
4-{4-[4-(Methylsulfonyl)phenoxy]phenyl}-2-(phenoxymethyl)-1H-imidazol;
4-{4-[2-(Phenoxymethyl)-1H-imidazol-4-yl]phenoxy}anilinchlorhydrat;
{4-[2-(Phenoxymethyl)-1H-imidazol-4-yl]phenyl}phenylmethanontrifluoracetat;
N-(4-Fluorphenyl)-4-[2-(phenoxymethyl)-1H-imidazol-4-yl]benzamidtrifluoracetat;
4-[4-(3-Nitrophenoxy)phenyl]-2-(phenoxymethyl)-1H-imidazol;
3-{4-[2-(Phenoxymethyl)-1H-imidazol-4-yl]phenoxy}anilinchlorhydrat;
4-{4-[4-(Benzyloxy)phenoxy]phenyl}-2-(phenoxymethyl)-1H-imidazol;
4-{4-[2-(Phenoxymethyl)-1H-imidazol-4-yl]phenoxy}phenol;
4-[4-(3-Fluorphenoxy)phenyl]-2-(phenoxymethyl)-1H-imidazol;
N-(4-{4-[2-(Phenoxymethyl)-1H-imidazol-4-yl]phenoxy}phenyl)acetamid;
2-Nitro-4-{4-[2-(phenoxymethyl)-1H-imidazol-4-yl]phenoxy}anilintrifluoracetat;
N-Methyl-N-(4-{4-[2-(phenoxymethyl)-1H-imidazol-4-yl]phenoxy}phenyl)amin;
3-{4-[2-(Phenoxymethyl)-1H-imidazol-4-yl]phenoxy}benzonitril;
4-[4-(2-Nitrophenoxy)phenyl]-2-(phenoxymethyl)-1H-imidazol;
2-{4-[2-(Phenoxymethyl)-1H-imidazol-4-yl]phenoxy}anilinchlorhydrat;
1-(4-{4-[2-(Phenoxymethyl)-1H-imidazol-4-yl]phenoxy}phenyl)methanaminchlorhydrat;
1-(3-{4-[2-(Phenoxymethyl)-1H-imidazol-4-yl]phenoxy}phenyl)methanaminchlorhydrat;
4-[4-(3-Bromphenoxy)phenyl]-2-(phenoxymethyl)-1H-imidazol;
2-Fluor-4-{4-[2-(phenoxymethyl)-1H-imidazol-4-yl]phenoxy}anilinchlorhydrat;
4-[4-(3-Chlorphenoxy)phenyl]-2-(phenoxymethyl)-1H-imidazol;
4-[4-(3,5-Difluorphenoxy)phenyl]-2-(phenoxymethyl)-1H-imidazol;
4-(4-Benzylphenyl)-2-(phenoxymethyl)-1H-imidazol;
4-[4-(3-Methylphenoxy)phenyl]-2-(phenoxymethyl)-1H-imidazol;
4-[4-(2-Chlorphenoxy)phenyl]-2-(phenoxymethyl)-1H-imidazolchlorhydrat;
4-[4-(2-Fluorphenoxy)phenyl]-2-(phenoxymethyl)-1H-imidazol;
4-[4-(3,4-Difluorphenoxy)phenyl]-2-(phenoxymethyl)-1H-imidazol;
N¹-(4-{4-[2-(Phenoxymethyl)-1H-imidazol-4-yl]phenoxy}phenyl)glycinamidchlorhydrat;
4-[4-(2,5-Difluorphenoxy)phenyl]-2-(phenoxymethyl)-1H-imidazol;
4-[4-(2,4-Difluorphenoxy)phenyl]-2-(phenoxymethyl)-1H-imidazol;
4-[4-(2,3-Difluorphenoxy)phenyl]-2-(phenoxymethyl)-1H-imidazol;
4-[4-(2,6-Difluorphenoxy)phenyl]-2-(phenoxymethyl)-1H-imidazol.

17. Verfahren zur Herstellung einer Verbindung der Formel (I) nach einem der vorhergehenden Ansprüche, **dadurch gekennzeichnet, dass** man eine Verbindung der Formel 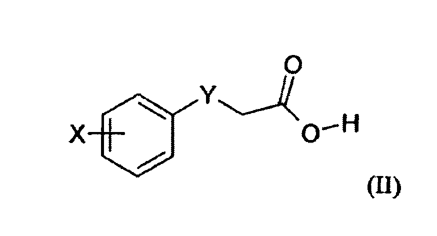 in der X und Y die oben angegebene Bedeutung haben, mit einer Base reagieren lässt, um die Verbindung (II) in einer Salzform zu bilden, und dann mit einem α-Halogenketon der Formel 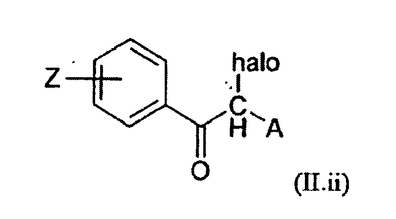 in der Z und A die oben angegebene Bedeutung haben, in einem inerten Lösungsmittel reagieren lässt,
und dann der auf diese Weise erhaltene Ketoester in Gegenwart eines Ammoniumsalzes cyclisiert wird, um die Verbindung der Formel (I) zu ergeben.

18. Verfahren zur Herstellung einer Verbindung der Formel (I) nach einem der Ansprüche 1 bis 16, **dadurch gekennzeichnet, dass** man eine Verbindung der Formel (II-iii) 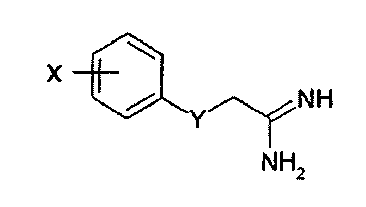 in der X und Y die in einem der vorhergehenden Ansprüche angegebene Bedeutung haben, und das α-Halogenketon der allgemeinen Formel (II-ii) 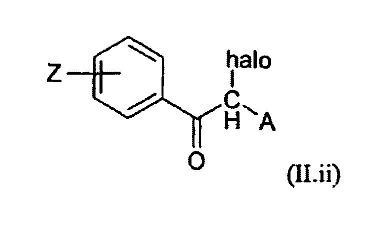 in der Z und A so wie in einem der vorhergehenden Ansprüche definiert sind, in einem polaren inerten Lösungsmittel unter Rückfluss kondensiert.

19. Pharmazeutische Zusammensetzung, die als Wirkstoff mindestens eine Verbindung nach einem der Ansprüche 1 bis 16 in Kombination mit einem pharmazeutisch annehmbaren Träger enthält.

20. Verwendung einer Verbindung nach einem der Ansprüche 1 bis 16 für die Herstellung eines antitumoralen Medikaments.

21. Verwendung einer Verbindung nach einem der Ansprüche 1 bis 16 für die Herstellung eines Medikaments, das zur Hemmung der Tubulin-Polymerisation bestimmt ist.

22. Verwendung einer Verbindung der Formel (I') 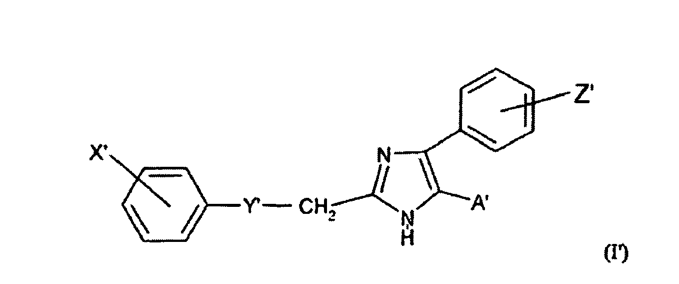 in racemischer Form, Enantiomerenform und allen Kombinationen dieser Formen, in der
X' einen oder mehrere gleiche oder verschiedene Substituenten darstellt, die aus H und Halogen ausgewählt sind;
Y' -0- oder -S- darstellt;
A' H oder (C₁-C₆)-Alkyl darstellt;
Z' einen oder mehrere gleiche oder verschiedene Substituenten darstellt, die ausgewählt sind aus:
. (C₁-C₆)-Alkyl, das gegebenenfalls mit einem oder mehreren gleichen oder verschiedenen Halogenresten substituiert ist;
. Aryl, das gegebenenfalls mit einem oder mehreren gleichen oder verschiedenen Resten substituiert ist, die ausgewählt sind aus: Halogen, Nitro, Cyano, Hydroxy, (C₁-C₆)-Alkyl, das gegebenenfalls mit einem oder mehreren gleichen oder verschiedenen Halogenresten substituiert ist, -(CH₂)ₙ-NR₃R₄, (C₁-C₆) -Alkylsulfonyl, (C₁-C₆)-Alkylthio, (C₁-C₆)-Alkoxy, das gegebenenfalls mit einem oder mehreren gleichen oder verschiedenen Halogenresten substituiert ist, (C₁-C₆)-Alkoxycarbonyl, Phosphat, Sulfat, Glycosid und -NH-C(O)-CH(R_{A})-NR₅R₆;
. Aryl-(C₁-C₆)-alkyl;
. Heteroaryl;
. -Z₁-Z'₁;
. -NR_{N}-C(O)-Z'₂; oder
. Z₂-Z'₂;
Z₁ -O-, -C(O)-O-, -NR_{N}-C(O)- oder -C(O)-NR_{N}- darstellt;
Z'₁ darstellt: einen Rest (C₁-C₁₀)-Alkyl; Aryl-(C₁-C₆)-alkyl, dessen Arylrest gegebenenfalls mit einem oder mehreren gleichen oder verschiedenen Halogenresten substituiert ist; oder (C₁-C₆)-Alkyl, das mit einem oder mehreren Substituenten substituiert ist, die ausgewählt sind aus: Halogen, (C₁-C₆)-Alkoxy, (C₁-C₆)-Alkylthio und -NR₁R₂;
R₁ und R₂ unabhängig voneinander H oder (C₁-C₆)-Alkyl darstellen oder zusammen mit dem Stickstoffatom, an das sie gebunden sind, ein Heterocycloalkyl bilden, das gegebenenfalls mit (C₁-C₆)-Alkyl substituiert ist;
Z₂ -O-, -S-, -SO₂-, -C(O)-, -C(O)-NR_{N}- oder -NR_{N}- darstellt;
Z'₂ einen Rest Aryl oder Heteroaryl darstellt, wobei die Reste Aryl und Heteroaryl gegebenenfalls mit einem oder mehreren gleichen oder verschiedenen Resten substituiert sind, die ausgewählt sind aus:
Halogen, Nitro, Cyano, Hydroxy, (C₁-C₆)-Alkyl, das gegebenenfalls mit einem oder mehreren gleichen oder verschiedenen Halogenresten substituiert ist, (C₁-C₆)-Alkylthio, (C₁-C₆) -Alkylsulfonyl, (C₁-C₆)-Alkoxy, das gegebenenfalls mit einem oder mehreren gleichen oder verschiedenen Halogenresten substituiert ist, Arylalkoxy, (C₁-C₆)-Alkoxycarbonyl, Phosphat, Sulfat, Glycosid, -(CH₂)ₙ-NR₃R₄ und -NH-C (O) -CH(R_{A}) -NR₅R₆;
R₃ und R₄ unabhängig voneinander H, (C₁-C₆)-Alkyl, (C₁-C₆)-Alkylcarbonyl oder (C₁-C₆)-Alkylsulfonyl darstellen oder R₃ und R₄ zusammen mit dem Stickstoffatom, an das sie gebunden sind, ein Heteroaryl oder ein Heterocycloalkyl bilden, das gegebenenfalls mit (C₁-C₆)-Alkyl substituiert ist;
R₅ und R₆ unabhängig voneinander H oder (C₁-C₆) -Alkyl darstellen;
R_{A} den mit der Aminosäure der Formel NH₂-CH(R_{A})-C(O)-OH assoziierten Rest darstellt;
R_{N} Wasserstoff oder einen (C₁-C₆)-Alkylrest darstellt;
n eine ganze Zahl von 0 bis 3 darstellt; oder ein pharmazeutisch annehmbares Salz dieser Verbindungen für die Herstellung eines antitumoralen Medikaments.

23. Verwendung einer Verbindung der Formel (I'), wie sie im Anspruch 22 definiert ist, für die Herstellung eines Medikaments, das zur Hemmung der Tubulin-Polymerisation bestimmt ist.
